# EUROPEAN PATENT APPLICATION

(11) **EP 4 516 204 A1**
(43) Date of publication of application: **05.03.2025**
(21) Application number: 24197239.7
(22) Date of filing: 29.08.2024
(51) Int. Cl.: A61B 3/032, A61B 3/08, A61B 3/09, A61B 3/103

(54) **OPHTHALMIC EXAMINATION APPARATUS AND OPHTHALMIC EXAMINATION METHOD**

(30) Priority: 31.08.2023 JP 2023141157
(71) Applicant: OSAKA UNIVERSITY, Suita-shi Osaka 565-0871 (JP); TOPCON CORPORATION, Tokyo 174-8580 (JP)
(72) Inventor: FUJIKADO, Takashi, Osaka, 565-0871 (JP); TATARA, Yoko, Tokyo, 174-8580 (JP); YUKIMORI, Takafumi, Tokyo, 174-8580 (JP); SAIKA, Makoto, Tokyo, 174-8580 (JP); NORO, Ryoka, Tokyo, 174-8580 (JP)
(74) Representative: Louis Pöhlau Lohrentz

(57) **Abstract**

An ophthalmic examination apparatus (100) includes a visual target projection system (4) including a visual target presentation portion (41) that presents a fixed visual target to left and right subject eyes (EL, ER), an objective measurement optical system (6, 7) that objectively measures eye characteristics of the left and right subject eyes, and a controller (140) that controls each portion of the apparatus, wherein the fixed visual target is a visual target for the left eye (8L) and a visual target for the right eye (8R) that are to be respectively projected to the left and right subject eyes (EL, ER) and have a same drawing pattern in positions where up and down direction positions to visual target flames (81L, 81R) are the same.

## Description

### CROSS-REFERENCE TO RELATED APPLICATION

The present application is based on and claims priority from Japanese Patent Application No. 2023-141157 filed on August 31, 2023, the disclosure of which is hereby incorporated by reference in its entirety.

### FILED OF THE INVENTION

The present disclosure relates to an ophthalmic examination apparatus and an ophthalmic examination method.

### BACKGROUND

Techniques regarding ophthalmic examinations have been disclosed in some documents such as JP 2022-038942 A, JP 2021-019957 A, JP 2020-069201 A, JP 2019-069049 A, JP 2019-063238 A, and JP 2018-047050 A. JP 2022-038942 A has disclosed a technique to provide optometry information that allows users to prescribe a correction lens for a subject (patient) with heterophoria. JP 2021-019957 A has described a technique to provide an ophthalmic measurement apparatus that allows users to examine eyes in a state closer to natural vision where the binocular fusion of the eye is appropriately maintained. JP 2020-069201 A has disclosed a technique to provide an optometry system that allows users to check the position of the line of sight of the subject eye and examine eyes more appropriately. JP 2019-069049 A has disclosed a technique to provide an ophthalmic apparatus that allows users to conduct an objective examination on a state related to an eye position. JP 2019-063238 A has disclosed a technique to provide an ophthalmic apparatus that allows users to conduct objective examination on a state related to an eye position. JP 2018-047050 A has disclosed a technique that allows uses to check a fusion state more easily of a subject who opens both eyes and measure the optical characteristics of the subject's eyes in higher accuracy.

### SUMMARY

When viewing a usual, three-dimensional (3D) video, eyes experience a discrepancy between convergence, which is the eye movement involved in fusing two images into both eyes as a single perception with both eyes and accommodation, which is an adjustment function involved in focusing the images with both eyes. That is, the convergence and the accommodation involve therebetween a range of the convergence (relative convergence) where the fusion of two visual targets into one with both eyes can be achieved while the accommodation is maintained constant accommodation, and a range of the accommodation (relative adjustment) where the fusion can be achieved while the convergence is maintained constant. For this reason, when exceeding the ranges of the relative convergence or the relative adjustment will trigger convergence movement (accommodation convergence) cooperating with the accommodative response or an accommodative reaction (convergence accommodation) cooperating with the convergence. Here, the term "convergence" refers to an eye movement of a subject (patient) in a direction in which both eyes approach each other, between eye movements in which both eyes move in different directions when the subject changes the line of sight from an object to another object at a different distance (depth direction to subject).

Meanwhile, with reference to JP 2022-038942 A, JP 2021-019957 A, JP 2020-069201 A, JP 2019-069049 A, or JP 2019-063238 A, these prior-art documents have disclosed no configuration for producing the discrepant state between the convergence and the accommodation by applying the convergence stimulus when the system arranges two visual targes at positions at a certain distance from the subject's eyes that sees the two visual targets fused with both eyes. For this reason, the prior-art disclosures fail to access the subject's eyes whether they can easily adapt themselves to stereoscopic vision, in which a discrepancy between convergence and accommodation occurs.

The present disclosure has been made in view of the above problems, and an object of the present disclosure aims to provide an ophthalmic examination apparatus and an ophthalmic examination method capable of evaluating whether the left and right subject eye can easily adapt themselves to stereoscopic vision in which a discrepancy between convergence and accommodation occurs.

An ophthalmic examination apparatus includes: a visual target projection system including a visual target presentation portion that presents a fixed visual target to left and right subject eyes; an objective measurement optical system that objectively measures eye characteristics of the left and right subject eyes; and a controller that controls each portion of the apparatus, wherein the fixed visual target is a visual target for the left eye and a visual target for the right eye that are to be respectively projected to the left and right subject eyes and have a same drawing pattern in positions where up and down direction positions to visual target flames are the same, the visual target presentation portion presents the visual target for the left eye and the visual target for the right eye to visual target positions that are separated from the left and right subject eyes by a certain examination distance, the visual target projection system prescribes a state in which a convergence distance from positions of the left and right subject eyes to a convergence position where two lines of sight intersect each other is changed by changing parallax that is difference in positions of the two drawing patterns reflected on retinas of the left and right subject eyes, and the controller measures the eye characteristics of the left and right subject eyes by the objective measurement optical system and acquires an objective refraction value as objective measurement information when fusion is attempted with the left and right subject eyes with respect to a change in the convergence distance.

An ophthalmic examination method in which a visual target projection system that includes a visual target presentation portion that presents a fixed visual target to left and right subject eyes, an objective measurement optical system that objectively measures eye characteristics of the left and right subject eyes, and a controller that controls each portion of apparatus are included, the method causes the controller to perform: a complete correction prescription step of assuming the left and right subject eyes as a state in which a complete correction value in subjective examination is prescribed; a visual target presentation step of presenting a visual target for the left eye and a visual target for the right eye having a same pattern in the visual target presentation portion at visual target positions separated from the left and right subject eyes by a certain examination distance following the prescription of the complete correction value; a convergence distance control step of prescribing a state in which a convergence distance from positions of the left and right subject eyes to a convergence position where the two lines of sight intersect each other is changed by control that changes parallax that is difference in positions of the two drawing patterns reflected on retinas of the left and right subject eyes; an accommodation reaction amount measurement step of being executed in parallel with the control of the convergence distance and measuring the eye characteristics of the left and right subject eyes by the objective measurement optical system when fusion is attempted with the left and right subject eyes to the change of the convergence distance to acquire an objective refraction value as objective measurement information; and a determination step of determining adaptation abilities of the left and right subject eyes to stereoscopic vision based on the objective refraction value.

### BRIEF DESCRIPTION OF DRAWINGS

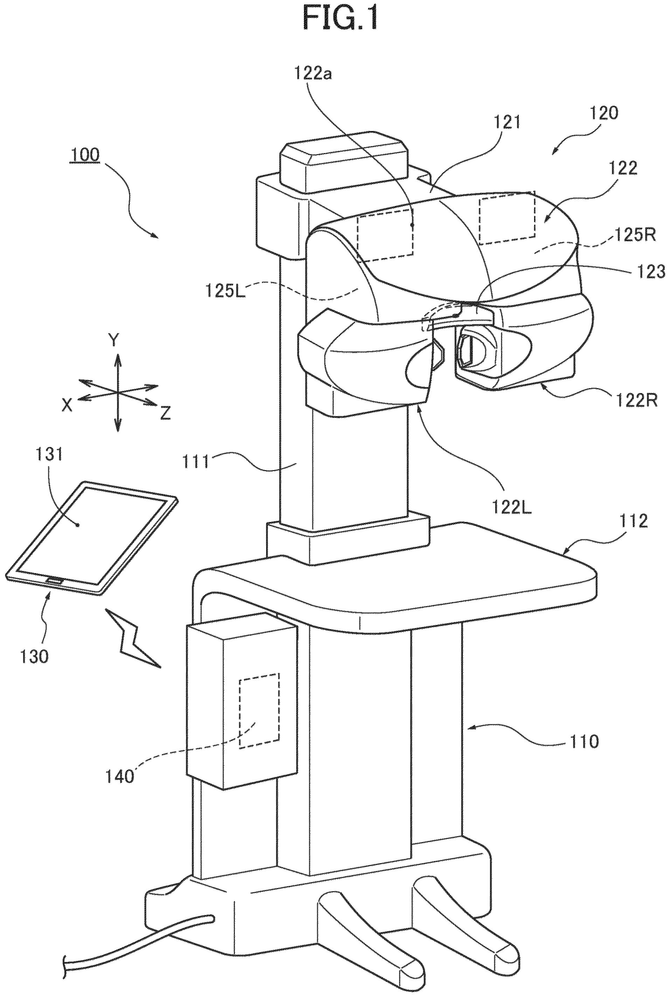

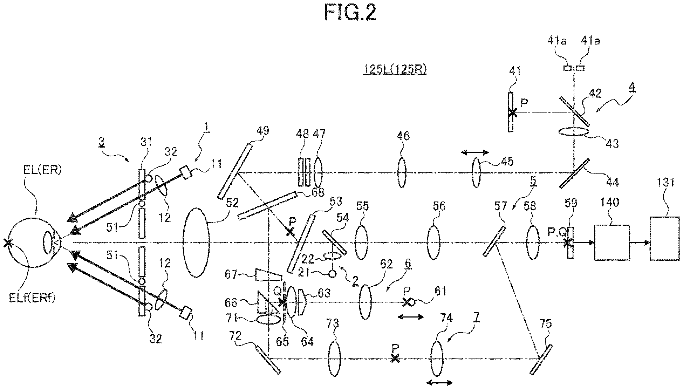

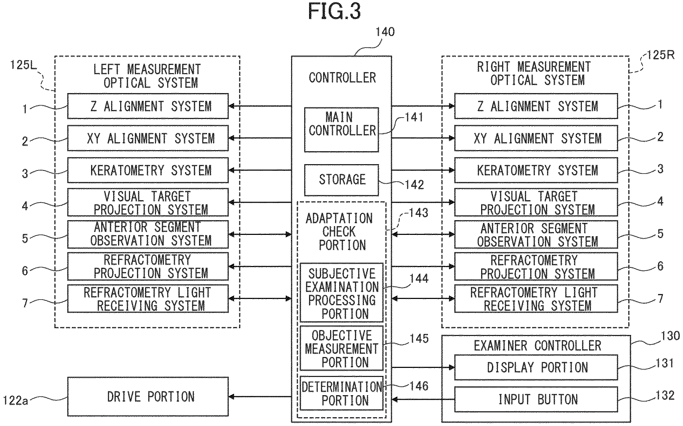

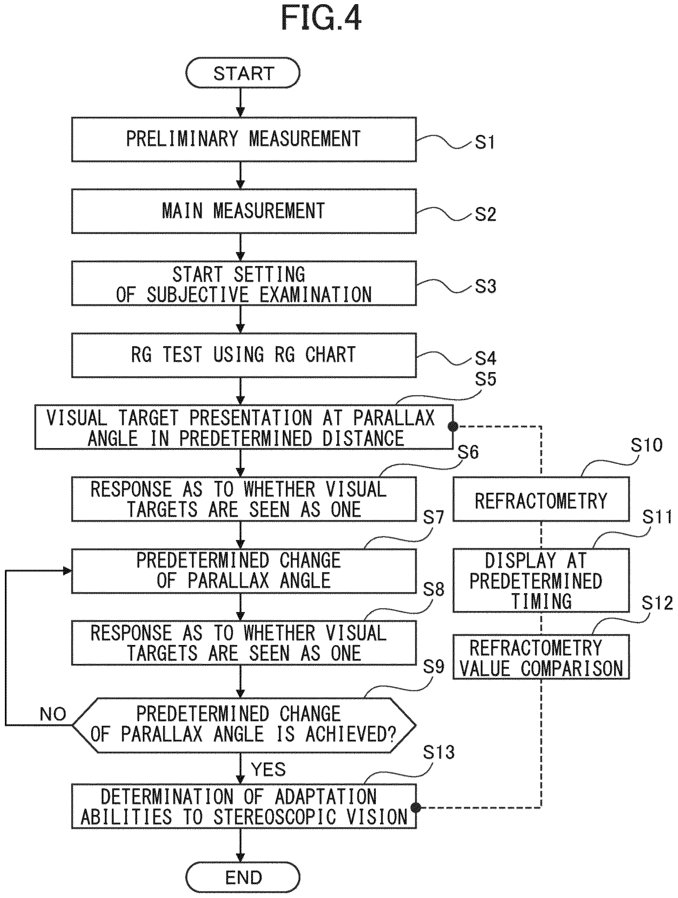

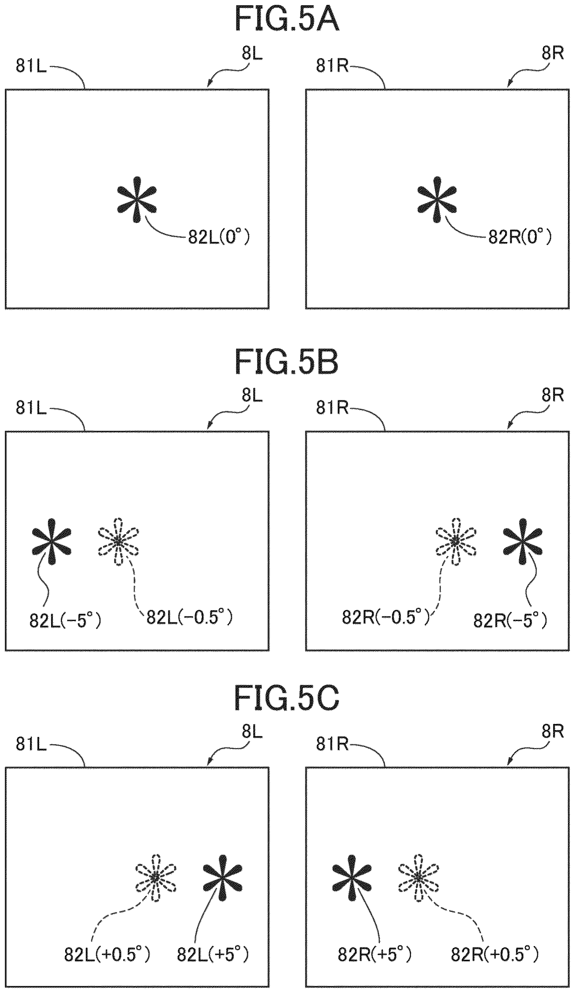

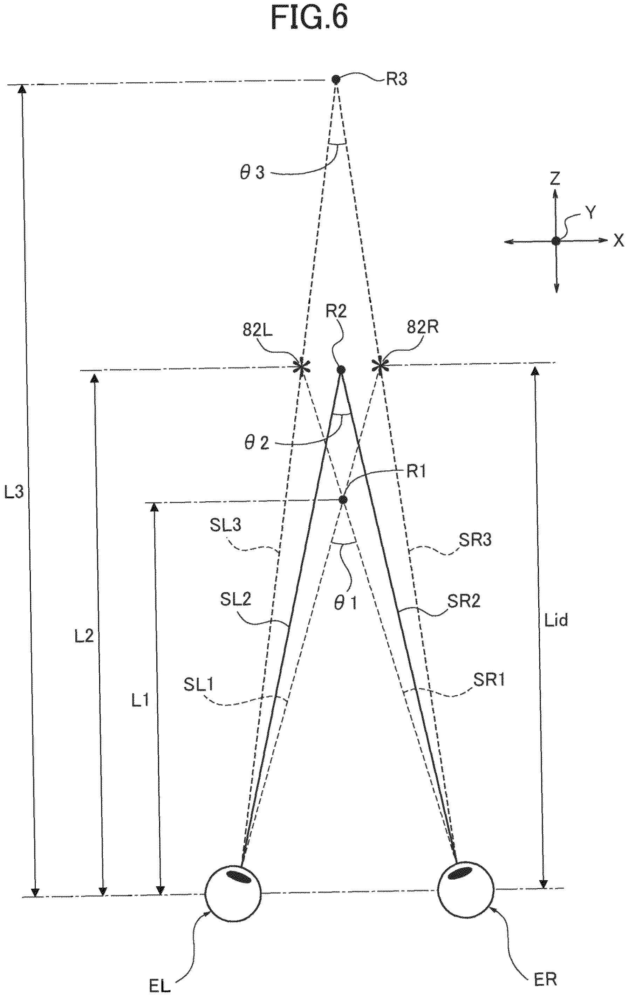

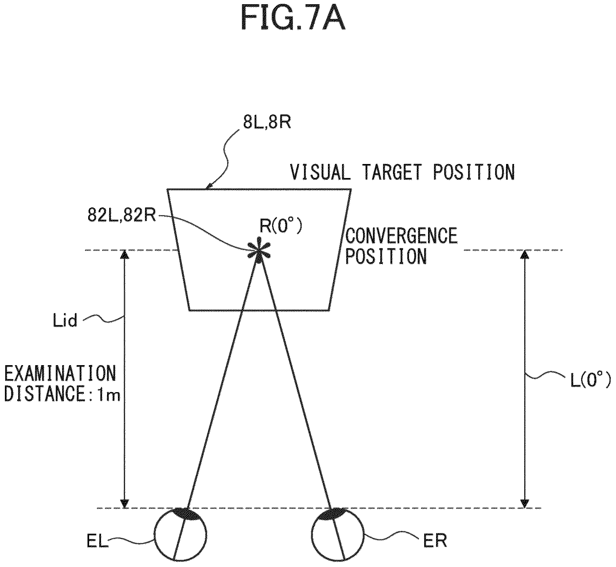

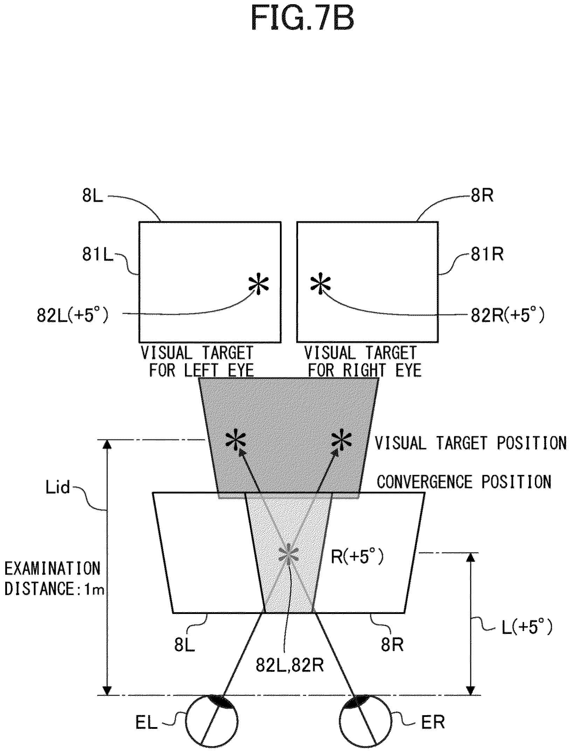

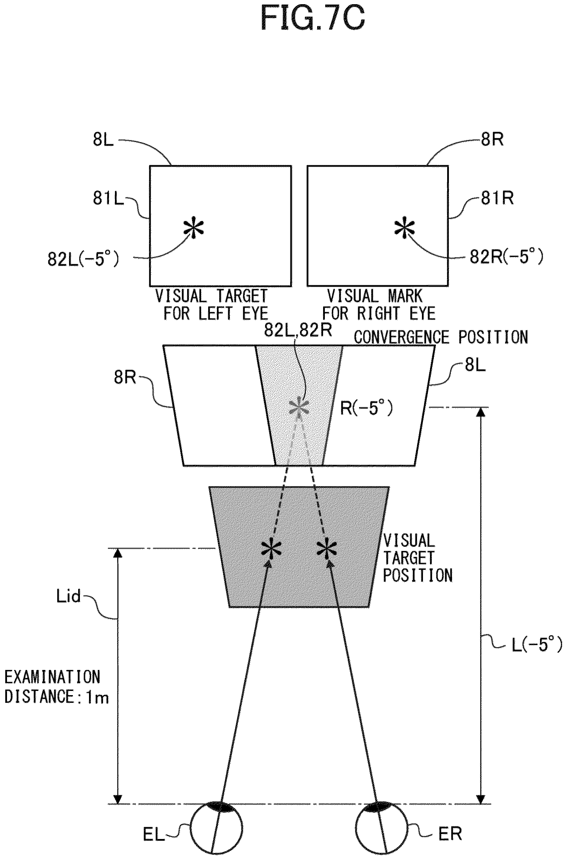

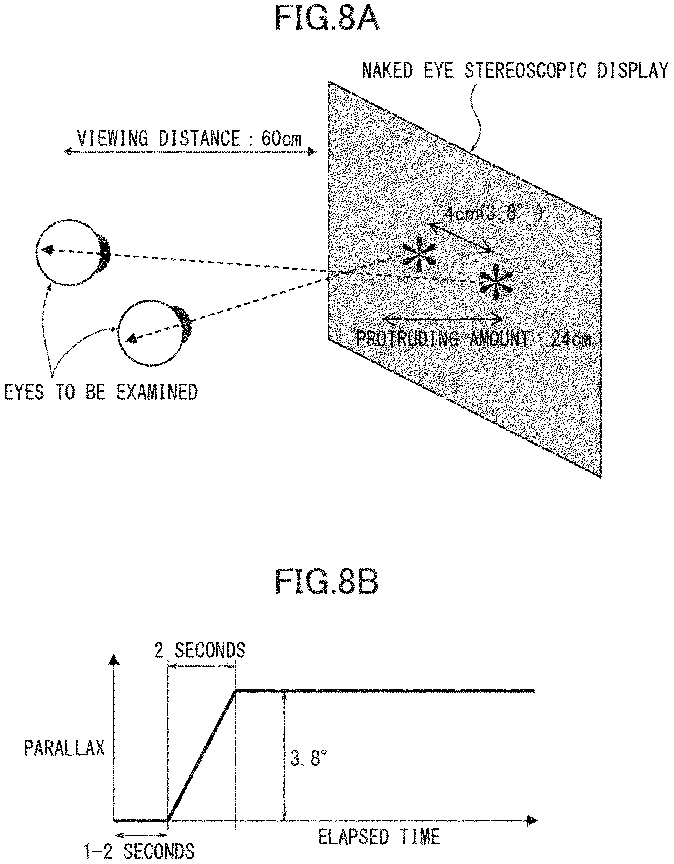

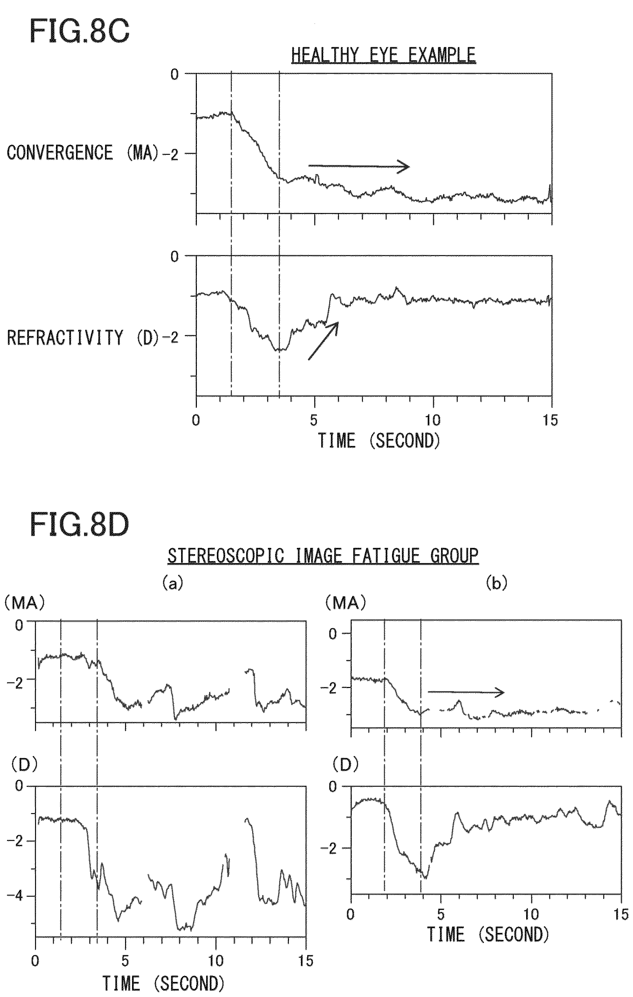

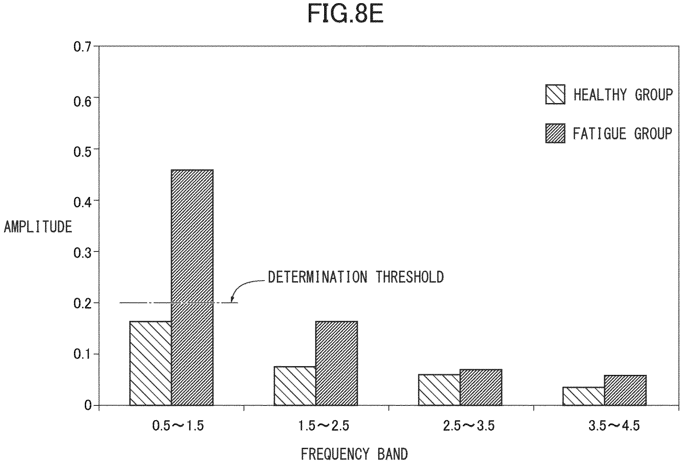

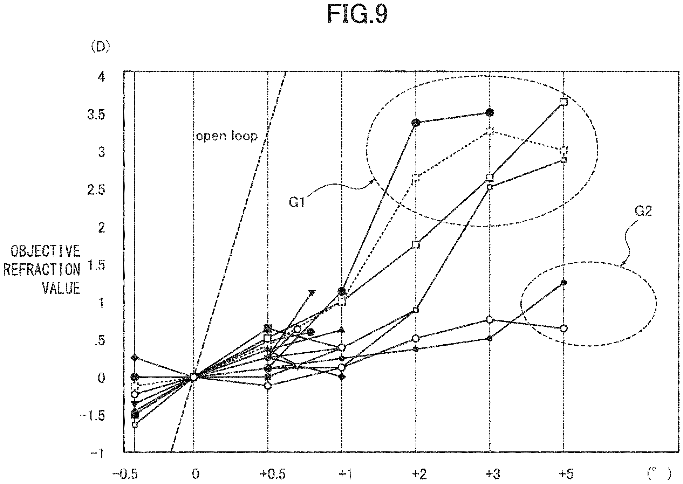

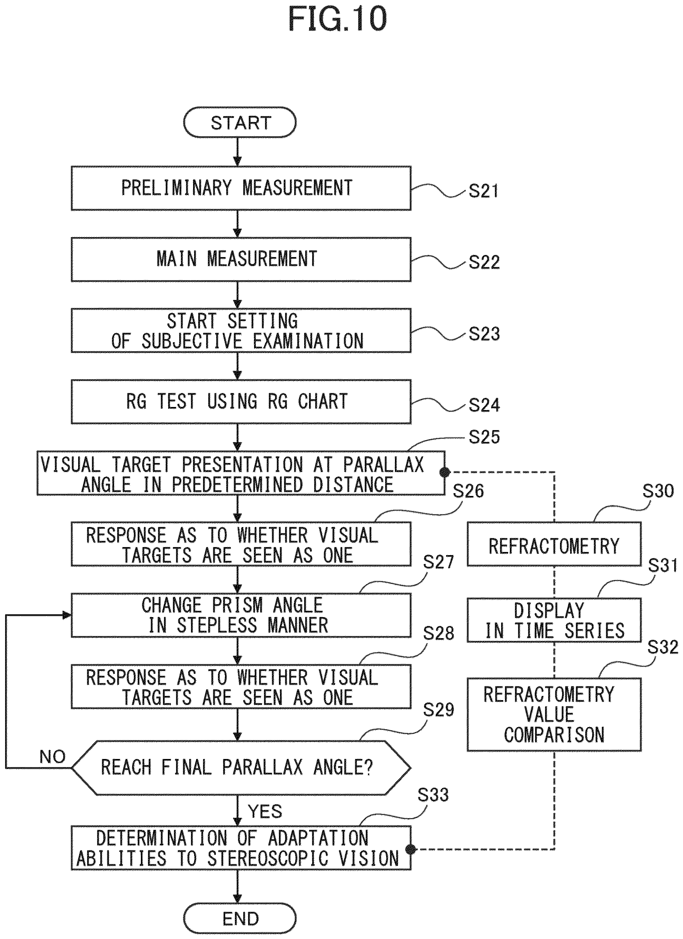

FIG. 1 is an external perspective view of an ophthalmic examination apparatus of Example 1. FIG. 2 illustrates a schematic configuration example of a left measurement optical system in the ophthalmic examination apparatus of Example 1. FIG. 3 illustrates a control block configuration of the ophthalmic examination apparatus according to Example 1. FIG. 4 is a flowchart showing a process sequence of an ophthalmic examination method of Example 1. FIG. 5A illustrates a pair of visual targets each including a drawing pattern position in a frame set in a position of an initial parallax angle. FIG. 5B illustrates a pair of a visual targets each including a drawing pattern set in a position in which a convergence distance is farther than an examination distance. FIG. 5C illustrates a pair of visual targets each including a drawing pattern set in a position in which a convergence distance is farther than an examination distance. FIG. 6 is an explanatory view illustrating a convergence distance to a convergence position where two lines of sight intersect each other. FIG. 7A illustrates an example in which the convergence distance to the convergence position matches with an examination distance. FIG. 7B illustrates an example in which the convergence distance to the convergence position is shorter than the examination distance. FIG. 7C illustrates an example in which the convergence distance to the convergence position is farther than the examination distance. FIG. 8A illustrates measurement of convergence and accommodation in observation of an stereoscopic image. FIG. 8B illustrates a parallax time chart in the measurement of the convergence and the accommodation. FIG. 8C illustrates measurement result of convergence and a refractivity (accommodation) by a fatigue group. FIG. 8D illustrates measurement result examples 1 and 2 of convergence and a refractivity (accommodation) by a fatigue group. FIG. 8E illustrates an analysis result example of a refraction time change frequency by a healthy group and a fatigue group. FIG. 9 illustrates a relationship characteristic between a parallax angle and an objective refraction value for which plurality of subject eye are measured. FIG. 10 is a flowchart showing a process sequence of an ophthalmic examination method of Example 2.

### DETAILED DESCRIPTION

With respect to the use of plural and/or singular terms herein, those having skill in the art can translate from the plural to the singular and/or from the singular to the plural as is appropriate to the context and/or application. The various singular/plural permutations may be expressly set forth herein for sake of clarity.

The embodiments for implementing an ophthalmic examination apparatus and an ophthalmic examination method of the present disclosure are described as follows based on Examples 1 and 2 shown in the drawings.

The ophthalmic examination apparatus of the Examples 1 and 2 is a both-eye open type apparatus that can simultaneously measure the eye characteristics of both eyes at the same time with an examinee opening both eyes. This ophthalmic examination apparatus can also measure the eye characteristics of each eye at a time by shielding one of the eyes or turning off a fixed visual target for the one of the eyes. In addition, the ophthalmic examination apparatus is an objective measurement machine with subjective examination functions and the objective measurement apparatus includes a visual target presentation function, a phoropter function, and an automatic refraction and keratometry function. This ophthalmologic apparatus allows an examiner to objectively and subjectively measure the eye characteristics of the subject eyes with arbitrary objective examination and arbitrary subjective examination.

The objective examination that the ophthalmic examination apparatus can execute includes measurement for acquiring the eye characteristics of the subject eye and photographing for acquiring the image of the subject eye. The objective examination includes the refractive power measurement (refractometry measurement), the corneal shape measurement (keratometry measurement), the intraocular pressure measurement, the ocular fundus photographing, the tomogram imaging using optical coherence tomography (OCT) (OCT imaging), and the measurement using OCT. In addition, the subjective examination presents, for example, a visual target to the subject, and measures information (eye characteristics) on the subject eye based on the response of the subject to the presented visual target, for example. The subjective examination includes subjective refraction measurement such as the far-point examination, the intermediate-point examination, the near-point examination, the contrast examination, and the glare examination, and the visual field examination.

### Example 1

[Overall Configuration of Apparatus (FIG. 1)] FIG. 1 describes an overall configuration of an ophthalmic examination apparatus 100. As illustrated in FIG. 1, the ophthalmic examination apparatus 100 includes a support base 110, a measurement unit 120, an examiner's control device examiner's control device 130, and a controller 140. Axes used in the drawings and the description, when viewed from the subject, includes an axis in the left-right direction (horizontal axis) as the X axis, an axis in the up-down direction (vertical axis) as the Y axis, and an axis perpendicular to the X axis and the Y axis in the depth direction (front-back axis) as the Z axis.

The support base 110 includes a support column 111 standing upright from the floor surface and an optometry table 112 supported by the support column 111. The optometry table 112 is a support on which apparatuses and tools used for optometry such as the examiner's control device 130 are placed or which supports the posture of the subject. The optometry table 112 may have a fixed position in the Y-axis direction (height position) or may be supported on the support column 111, which can adjust the position in the Y-axis direction (height position).

The measurement unit 120 includes an arm 121, a measurement head 122, and a forehead rest 123. The arm 121 includes one end supported by the distal end portion of the support column 111, the other end extending from the support column 111 to the front side (subject side) in the Z-axis direction, and the distal end portion to which the measurement head 122 is attached. As a result, the measurement head 122 suspends suspended from the support column 111 via the arm 121 above the optometry table 112. The arm 121 is also configured to move in the Y-axis direction with respect to the support column 111. Note that the arm 121 is also configured to move in the X-axis direction or the Z-axis direction with respect to the support column 111.

The measurement head 122 measures the eye characteristics of the subject eye. The measurement head 122 includes a drive portion 122a, a pair of a left measurement head portion 122L and a right measurement head portion 122R provided below the drive portion 122a, and the forehead rest 123. Here, the left measurement head portion 122L and the right measurement head portion 122R individually correspond to the left and right subject eye of the subject.

The drive portion 122a is a mechanism that individually drives each of the left measurement head portion 122L and the right measurement head portion 122R to move in the horizontal direction (X-axis direction), move in the vertical direction (Y-axis direction), rotate about the X-axis, or rotate about the Y-axis.

The left measurement head portion 122L includes a left measurement optical system 125L. The left measurement optical system 125L presents a visual target to the subject eye on the left side of the subject (hereinafter, "left subject eye") and measures eye characteristics of the left subject eye in a state where an arbitrary spherical power (correction degree) is set. The right measurement head portion 122R includes a right measurement optical system 125R. The right measurement optical system 125R presents a visual target to a subject eye on the right side of the subject (hereinafter, "right subject eye") and measures eye characteristics of the right subject eye in a state where an arbitrary spherical power (correction degree) is set. The measurement results by the left measurement optical system 125L and the right measurement optical system 125R are hereinafter referred to as objective measurement information. Note that detailed configurations of the left measurement optical system 125L and the right measurement optical system 125R are described below in "Configuration of Optical System".

The forehead rest 123 is provided in the measurement unit 120 and is disposed between the left measurement head portion 122L and the right measurement head portion 122R. The forehead rest 123 supports the face of the subject by bringing a part (forehead) of the face of the subject into contact during the measurement of the eye characteristics. That is, the subject facing the optometry table 112 presses the subject's own forehead against the forehead rest 123 to stabilize the orientation and position of the face so as not to move. Moving the arm 121 in the Y-axis direction with respect to the support column 111 adjusts the position of the forehead rest 123 in the height direction.

The examiner's control device 130 is an information processing device that receives an input operation by the examiner and outputs a control signal to the controller 140. The examiner's control device 130 is, for example, a tablet terminal and a smartphone and is separated from the measurement unit 120 and is portable by the examiner. Note that the examiner's control device 130 may be a notebook personal computer or a desktop personal computer or may be a dedicated controller provided in the ophthalmic examination apparatus 100. The examiner's control device 130 exchanges information with the controller 140 via wireless communication or network communication.

As illustrated in FIG. 1, the examiner's control device 130 includes a display portion 131 and an operation-side controller (not illustrated). The display portion 131 includes a touch panel display provided on the surface of the examiner's control device 130, and, for example, screen display sets an input button 132 (see FIG. 3). The operation-side controller includes a microcomputer equipped in the examiner's control device 130. The operation-side controller controls an image to be displayed on the display portion 131 based on a measurement result and a detection result transmitted from the controller 140. Further, the operation-side controller outputs a control signal corresponding to an operation on the displayed input button 132, for example, to the controller 140.

[Configuration of Optical System (FIG. 2)] Detailed configurations of the left measurement optical system 125L and the right measurement optical system 125R are described below with reference to FIG. 2. Note that the left measurement optical system 125L and the right measurement optical system 125R have the same configuration. Accordingly, on the present specification, the illustration and the description of the right measurement optical system 125R corresponding to a right subject eye ER are omitted, and only the left measurement optical system 125L corresponding to a left subject eye EL is described.

The left measurement optical system 125L is an optical system that presents a visual target to the left subject eye EL and measures the left subject eye. As illustrated in FIG. 2, the left measurement optical system 125L includes a Z alignment system 1, an XY alignment system 2, a keratometry system 3, a visual target projection system 4, an anterior segment observation system 5, a refractometry projection system 6, and a refractometry light receiving system 7. Note that a "fundus conjugate position P" used in the following description is a position optically substantially conjugate with a fundus ELf of the left subject eye EL when alignment is completed and means a position optically conjugate with the fundus ELf of the left subject eye EL or the vicinity thereof. A "pupil conjugate position Q" is a position optically substantially conjugate with the pupil of the left subject eye EL when alignment is completed and means a position optically conjugate with the pupil of the left subject eye EL or the vicinity thereof.

The Z-alignment system 1 projects light (infrared light) for aligning in the optical axis direction (front-back direction = Z-axis direction) of the anterior segment observation system 5 to the left subject eye EL. The light projected from a Z-alignment light source 11 is converted into a parallel luminous flux by using a projection lens 12 and projected onto the cornea of the left subject eye EL through an alignment hole formed in a keratometric plate 31. Based on the bright spot projected on the cornea, the controller 140 or the examiner controls the drive portion 122a so that a ratio between a distance between the two-point images by the Z alignment light source 11 on an imaging element 59 and the diameter of a keratometric ring image falls within a predetermined range and moves the left measurement optical system 125L in the Z-axis direction.

The XY-alignment system 2 irradiates the left subject eye EL with light (infrared light) for performing alignment in the X-axis direction and the Y-axis direction orthogonal to the optical axis (Z-axis) of the anterior segment observation system 5. The XY-alignment system 2 includes an XY-alignment light source 21 and a projection lens 22 which are disposed in an optical path that is branched from that of the anterior segment observation system 5 with a half mirror 54. The light output from the XY alignment light source 21 transmits through the projection lens 22, reflects with the half mirror 54, and projects to the left subject eye EL through the anterior segment observation system 5. Reflected light from the cornea of the left subject eye EL is guided to the imaging element 59 through the anterior segment observation system 5. Note that the alignment method in each of XYZ-directions is not limited to the method using the Z-alignment system 1 or the XY-alignment system 2, and any method may be used as long as the position of the subject eye E can be measured, such as a method using a stereo camera for the alignment provided with the ophthalmic examination apparatus 100.

Here, the reflected light from the cornea of the left subject eye EL forms an image on the imaging element 59 as a bright spot image, which is included in a part of an anterior segment image. The controller 140 controls the display portion 131 to display the anterior segment image, which includes the bright spot image, and an alignment mark The anterior segment image (bright spot image) and alignment mark are used as follows: for the manual XY-alignment, the examiner uses the examiner's control device 130 to control the drive portion 122a to move the left measurement optical system 125L in the X-axis direction and the Y-axis direction so as to put the bright spot image in the alignment mark; for the automatic XY alignment, the controller 140 controls the drive portion 122a to move the left measurement optical system 125L in the X-axis direction and the Y-axis direction so as to eliminate the displacement of the bright spot image relative to the alignment mark.

The keratometry system 3 projects a ring-shaped luminous flux (infrared light) onto the cornea to measure the shape of the cornea of the left subject eye EL onto the cornea. The keratometric plate 31 is disposed at a position between an objective lens 52 and the left subject eye EL, and a keratometric ring light source 32 is provided on the back surface side (objective lens 52 side). The keratometric ring light source 32 projects a ring-shaped luminous flux onto the cornea of the left subject eye EL by illuminating the keratometric plate 31 with light from the back surface. Reflected light (keratometric ring image) from the cornea of the left subject eye EL is detected as well as the anterior segment image with the imaging element 59. The controller 140 calculates corneal shape parameters representing the shape of the cornea with known calculation based on the keratometric ring image.

The visual target projection system 4 presents various visual targets such as a fixed visual target and a visual target for the subjective examination, to the left subject eye EL. The visual target projection system 4 includes a display 41, a half mirror 42, a relay lens 43, a reflection mirror 44, a focusing lens 45, a relay lens 46, a field lens 47, a variable cross cylinder lens (VCC) 48, and a reflection mirror 49. The visual target projection system 4 shares a dichroic mirror 68 with the refractometry projection system 6. The visual target projection system 4 shares a dichroic mirror 53 and the objective lens 52 with the anterior segment observation system 5. Furthermore, the visual target projection system 4 includes at least two glare light sources 41a, which illuminates the left subject eye EL with glare light, at a position around the optical axis and on an optical path different from an optical path to the display 41, which displays the visual target for the left eye.

The light projected from the display 41 reflects on the half mirror 42, transmits through the relay lens 43, reflects on the reflection mirror 44, and transmits through the focusing lens 45. The light transmitted through the focusing lens 45 transmits through the relay lens 46, transmits through the VCC 48 after the filed lens 47 aligns traveling direction, reflects on the reflection mirror 49, transmits through the dichroic mirror 68, and reflects on the dichroic mirror 53. The light reflected with the dichroic mirror passes through the objective lens 52 and is projected onto the fundus ELf.

The display 41 is configured to display various visual targets including a fixed visual target or a point visual target as a visual target for fixing a line of sight for an objective examination or for cloud fogging to the left subject eye EL, and a subjective examination visual target for subjectively examining eye characteristics (visual acuity value, dioptric power for long distance, dioptric power for short distance, and the like) of the left subject eye EL. The display 41 may be fabricated with a liquid crystal display or an organic EL display. Then, the visual targets to be projected on the subject eye E can be arbitrarily created by using a still image or a moving image, which can be used as a chart icon that can be selected on a chart page. The display 41 is configured to display a selected created visual target of the still image or a created visual target of the moving image from the chart page. The display 41 is provided at the fundus conjugate position P on the optical path of the visual target projection system 4.

The focusing lens 45 is moved forward and backward in the optical axis direction by a drive motor (not illustrated) controlled by the controller 140. Controlling to move the focusing lens 45 in a direction toward the left subject eye EL allows the controller 140 to change the spherical power of the left subject eye EL to the negative diopter side (-D side). Also, controlling to move the focusing lens 45 in a direction away from the left subject eye EL allows the controller 140 to change the spherical power of the left subject eye EL to the positive diopter side (+D side). Furthermore, controlling to move the focusing lens 45 forward and backward allows the controller 140 to change the examination distance between the left subject eye EL and the visual target presentation position. Here, with the controlling of the focusing lens 45, a refractometry light source 61, and a focusing lens 74 are controlled to move together.

For the subjective examination, the controller 140 controls the examination distance or the spherical power of the left subject eye EL by moving the focusing lens 45 in the optical axis direction based on the result of the objective measurement. Then, the controller 140 displays a predetermined visual target selected by the examiner or the controller 140 on the display 41. As a result, the visual target is presented to the subject at a predetermined examination distance with respect to the left subject eye EL adjusted to a predetermined spherical power. Also, the controller 140 is configured to receive a subjective response the subject has made to the visual target. For example, for visual acuity measurement, the examiner or the controller 140 is configured to present the visual target, such as the Landolt's ring, to the subject and receive the subjective response to select the alternative mark, which are conducted repeatedly, to determine the visual acuity value of the subject.

The anterior segment observation system 5 observes the anterior segment of the left subject eye EL and captures the anterior segment. An anterior segment illumination light source 51 illuminates light (for example, infrared light) onto the anterior segment of the left subject eye EL. The light reflected on the anterior segment of the left subject eye EL passes through the objective lens 52, transmits through the dichroic mirror 53, transmits through the half mirror 54, passes through a relay lens 55 and a relay lens 56, and transmits through a dichroic mirror 57. The light transmitted through the dichroic mirror 57 forms an image on the imaging surface of the imaging element 59 by using an image forming lens 58. The imaging element 59 captures to output a signal at a predetermined rate. The output (video signal) of the imaging element 59 is input to the controller 140. The controller 140 displays an anterior segment image (moving image), which is based on the image signal as an output of the imaging element 59, on the display portion 131 of the examiner's control device 130. The imaging surface of the imaging element 59 in the optical system of the anterior segment observation system 5 is arranged at the pupil conjugate position Q.

The refractometry projection system 6 and the refractometry light receiving system 7 constitute objective measurement optical systems used for objective refraction measurement (measurement) that objective measure a refraction value as eye characteristics of the left subject eye EL. Hereinafter, the refractometry projection system 6 and the refractometry light receiving system 7 are referred to as the objective measurement optical systems 6 and 7. The refractometry projection system 6 projects a ring-shaped luminous flux (infrared light) for objective measurement from the refractometry light source 61 onto the fundus ELf. The refractometry light receiving system 7 receives the return light of the ring-shaped luminous flux from the left subject eye EL.

The refractometry light source 61 may be fabricated with a super luminescent diode (SLD) light source, which is a high luminance light source with a light emission diameter less than a predetermined size. The refractometry light source 61 is configured to move in the optical axis direction together with the focusing lens 45 and the focusing lens 74 and is disposed at the fundus conjugate position P. A ring aperture 65 (specifically, the light transmitting portion) is disposed at the pupil conjugate position Q. The focusing lens 74 is configured to move in the optical axis direction together with the refractometry light source 61 and the focusing lens 45. The focusing lens 74 may be fabricated with a known variable focus lens, which can change the focal position under the control of the controller 140. The imaging surface of the imaging element 59 in the optical system of the refractometry light receiving system 7 is disposed at the fundus conjugate position P.

The light projected from the refractometry light source 61 passes through a relay lens 62 and enters onto the conical surface of a conical prism 63. The light incident onto the conical surface is deflected and emitted from the bottom surface of the conical prism 63. The light emitted from the bottom surface of the conical prism 63 passes through a field lens 64 and passes through a light transmitting portion formed in a ring shape in the ring aperture 65. The light (ring-shaped luminous flux) passing through the light transmitting portion of the ring aperture 65 reflects on the reflection surface of an aperture prism 66, passes through a rotary prism 67, and reflects on the dichroic mirror 68. The light reflected with the dichroic mirror 68 reflects on the dichroic mirror 53, passes through the objective lens 52, and is projected onto the left subject eye EL.

The conical prism 63 is disposed at a position as close as possible to the pupil conjugate position Q desirably. The conical prism 63 may have the ring aperture 65 attached to its bottom surface facing at the field lens 64. In this case, for example, the bottom surface of the conical prism 63 may be deposited with a light shielding film so as to form a ring-shaped light transmitting portion. Alternatively, the ring aperture 65 may be on the conical surface side of the conical prism 63.

The field lens 64 has a lens surface on the side of the left subject eye EL. For example, the ring aperture 65 may be attached to the lens surface of the field lens 64. In this case, for example, the lens surface of the filed lens 64 may be deposited with a light shielding film so as to form a ring-shaped light transmitting portion. Note that the refractometry projection system 6 may also be configured without the field lens 64. The ring aperture 65 may be formed with the light transmitting portion having a shape corresponding to a predetermined measurement pattern, at a position eccentric to (offset from) the optical axis of the refractometry projection system 6. The number of the light transmitting portions may be one or more than two. The rotary prism 67 is used for averaging the light amount distribution of the ring-shaped luminous flux with respect to the blood vessel and the disease site of the fundus ELf and reducing speckle noise caused by the light source.

The return light of the ring-shaped luminous flux projected on the fundus ELf passes through the objective lens 52 and reflects on the dichroic mirror 53 and the dichroic mirror 68. The return light reflected by the dichroic mirror 68 passes through the rotary prism 67, passes through the hole portion of the aperture prism 66, passes through a relay lens 71, reflects on a reflection mirror 72, and passes through a relay lens 73 and the focusing lens 74. The light passing through the focusing lens 74 reflects on a reflection mirror 75, reflects on the dichroic mirror 57, and forms an image on the imaging surface of the imaging element 59 by using the image forming lens 58.

The controller 140 calculates the parameters of the eye refraction power by a known calculation based on the output from the imaging element 59. Note that the parameters of the eye refraction power include the refraction values (refractive power), the spherical power, the astigmatism power, and the astigmatism axis angle of the left and right subject eye EL and ER. Based on the control signal transmitted from the examiner's control device 130, the controller 140 integrally controls each portion of the measurement unit 120, which includes the drive portion 122a and the left and right measurement optical systems 125L, 125R, each of which includes the refractometry projection system 6, the refractometry light receiving system 7, the visual target projection system 4. In addition, the controller 140 transmits the measurement results regarding the eye characteristics of the left subject eye EL and the right subject eye ER, which have been measured by using the measurement head 122, to the examiner's control device 130.

[Configuration of Controller (FIG. 3)] FIG. 3 describes the configuration of the controller 140 that totally controls each portion of the apparatus. As illustrated in FIG. 3, the controller 140 includes a main controller 141, a storage 142, and an adaptation check portion adaptation check portion 143.

The main controller 141 controls light-amount accommodation and the on-and-off for various light sources including the Z-alignment light source 11 of the Z-alignment system 1, the XY-alignment light source 21 of the XY-alignment system 2, and the keratometric ring light source 32 of the keratometry system 3. The main controller 141 controls on/off of the visual target, which is displayed on the display 41 of the visual target projection system 4 and changing of the visual target. The main controller 141 controls the light-amount accommodation and the on-and-off switching for the anterior segment illumination light source 51 of the anterior segment observation system 5. The main controller 141 controls the exposure time and detection sensitivity accommodation for the imaging element 59 of the anterior segment observation system 5. The main controller 141 controls light-amount accommodation and the on-and-off switching for the refractometry light source 61 of the refractometry projection system 6. The main controller 141 controls rotation speed accommodation for the rotary prism 67 of the refractometry projection system 6 and the on-and-off switching for the rotation operation. In addition, the main controller 141 controls movement to move the focusing lens 45 of the visual target projection system 4, the refractometry light source 61 of the refractometry projection system 6, and the focusing lens 74 of the refractometry light receiving system 7 in the optical axis direction in an associated manner. Note that the main controller 141 reads and writes data from and into the storage 142.

The storage 142 stores various data. The data stored in the storage 142 include the measurement information acquired with the keratometry system 3, the measurement information acquired with the objective measurement optical systems 6 and 7, the image data acquired with the imaging element 59, and the subject eye information. The information of the subject eye includes information regarding the subject such as an ID and a name, and information regarding the left and right subject eyes EL and ER such as identification information of the left eye and the right eye. The storage 142 stores the measurement information acquired with the keratometry system 3 when the ophthalmologic apparatus 100 performs the keratometry measurement of the left and right subject eye EL and ER. The storage 142 stores the measurement information acquired with the objective measurement optical systems 6 and 7 when the ophthalmologic apparatus 100 performs the refractometry measurement of the left and right subject eye EL and ER. The storage 142 may be used as a work memory of a calculation process of a cornea shape parameter and a calculation process of a refraction value of the left and right subject eye EL and ER. The storage 142 stores various programs and data for operating the ophthalmic examination apparatus 100.

The adaptation check portion 143 accesses the adaptation abilities to stereoscopic vision of the left and right subject eye EL and ER. The adaptation check portion 143 includes a subjective examination processing portion 144, an objective measurement portion 145, and a determination portion 146.

The subjective examination processing portion 144 assumes the left and right subject eye EL and ER into a state in which the complete correction value in the subjective examination is prescribed and presents the visual target for the left eye and the visual target for the right eye at visual target positions separated from the left and right subject eye EL and ER by a certain examination distance (for example, 1 m), respectively. The subjective examination processing portion 144 sets the visual target for the left eye and the visual target for the right eye on the display 41 (visual target presentation portion) that presents fixed visual targets to the left and right subject eyes EL and ER included in the visual target projection system 4. The left eye projection visual target and the right eye projection visual target, which projected onto the left and right subject eyes EL and ER, respectively, have the same drawing pattern in positions where up and down direction positions to a visual target frame are the same, and fuses the two drawing patterns by the right and left subject eye.

The subjective examination processing portion 144 is configured to change a convergence distance, which is a distance from the positions of the left and right subject eyes EL and ER to the convergence position where the two lines of sight of the each eye converge each other, by the control that changes the parallax, which is the difference in the positions of the two drawing patterns reflected on the retinas of the left and right subject eye EL and ER. The visual target for the left eye and the visual target for the right eye have the displayed drawing patterns, respectively, at the different positions in the right and left directions to set to stereoscopic visual targets provided with parallax by differentiating positions of the two drawing patterns in the right and left directions to the visual target frame. A plural pairs visual target for the left eye and the visual target for the right eye with the parallax are then prepared for different parallax. The subjective examination processing portion 144 uses the control that changes the parallax as the control that changes the parallax angle when the fusion of the two drawing patterns by binocular vision are attempted. The control that changes the parallax is performed by the control that switches a pair of visual targets displayed on the display 41 of the visual target projection system 4 in the main controller 141 based on the command from the subjective examination processing portion 144.

The subjective examination processing portion 144 is configured to change the convergence distance in a stepwise manner in the distance range from a position farther than the examination distance to a position closer than the examination distance by stepwisely changing the parallax which is the difference in the positions of the two drawing patterns reflected on the retinas of the left and right subject eye EL and ER. The subjective examination processing portion 144 acquires a subjective response from the subject when the left and right subject eyes EL and ER attempt the fusion with respect to the stepwise change in the convergence distance.

The objective measurement portion 145 executes processes in parallel with the process in the subjective examination processing portion 144. The objective measurement portion 145 measures the eye characteristics of the left and right subject eye EL and ER by using the objective measurement optical systems 6 and 7 and acquires the objective refraction values as the objective measurement information when the left and right subject eye EL and ER attempt the fusion with respect to the changes in the convergence distance. Here, the process in the subjective examination processing portion 144 is a process of changing the convergence distance in a stepwise manner. Therefore, the objective measurement portion 145 measures the characteristics of the left and right subject eye EL and ER a predetermined number of times at each stage of the changing convergence distance and averages objective refraction values obtained a predetermined number of times in each step to acquire as the objective measurement information, the objective refraction average value.

The determination portion 146 determines the adaptive ability of the left and right subject eye EL and ER with respect to the stereoscopic vision based on the subjective responses from the subject obtained with the subjective examination processing portion 144 and the objective refraction values (reflex values) acquired with the objective measurement portion 145. The determination portion 146 determines that the left and right subject eye EL, ER have high adaptation ability to the stereoscopic vision when the subject's response of the subjective examination is positive regardless of the change of the convergence distance, and when the among of the objective refraction value regardless of the change of the convergence distance is suppressed in a predetermined range. The determination portion 146 determines that the left and right subject eye EL and ER have low adaptation ability to the stereoscopic vision when the subject's responses of the subjective examination are negative s if the convergence distance is changed, and when the amount of the objective refraction value if the convergence distance is changed exceeds the predetermined range.

[Procedure of Evaluating Adaptation Abilities to Stereoscopic Vision (FIG. 4)] The configuration and flow of actions of the process of evaluating the adaptation abilities to stereoscopic vision by using the adaptation check portion 143 are described as follows with reference to the flowchart shown in FIG. 4. FIG. 4 illustrates an example of the operation of the adaptation check portion 143.

Step S1 is a preliminary measurement step in which the main controller 141 acquires the eye characteristics including the refraction values of the left and right subject eye EL and ER based on the captured image of the ring-shaped image, for example. Note that the main controller 141 may execute the preliminary measurement may be executed twice or more to determine the measurement conditions of the same type or those of different types by executing each of the preliminary measurement so that the measurement conditions should be converged each time while the preliminary measurement is repeated. For example, preliminary measurement each time determines, based on the obtained ring-shaped image, at least one of the light amount from the refractometry light source 61, the exposure time of the imaging element 59, the detection sensitivity of the imaging element 59, and the control content for the focusing lens 74.

Step S2 is a main measurement step in which the main controller 141 measures the objective refraction values of the target left and right subject eyes EL and ER under the measurement conditions determined by preliminary measurement executed the last time. Note that the objective refraction values of the left and right subject eyes EL and ER are measured by the following sequence. That is, the main controller 141 projects a ring-shaped luminous flux (infrared light) for objective measurement onto the fundus ELf and ERf of the left and right subject eyes EL and ER with the refractometry projection system 6. Then, the main controller 141 detects (receives an image of) the return light of the ring-shaped luminous flux from the fundus ELf and ERf by the refractometry light receiving system 7 and acquires the objective refraction values of the left and right subject eyes EL and ER by a known method based on the image signal from the imaging element 59.

Step S3 is a start setting step of the subjective examination in which the main controller 141 sets the start of the subjective examination for each of the left and right subject eyes EL and ER. Here, the "start setting of the subjective examination" means that the position of the focusing lens 45 in the visual target projection system 4 is adjusted based on the refraction values of the left and right subject eyes EL and ER obtained in the main measurement such that the spherical power of the left and right subject eyes EL and ER is in the complete correction state at the examination distance at the distant position.

Step S4 is an RG test step in which the main controller 141 performs an RG test using an RG chart. Here, the "RG test" refers to an examination for subjectively checking whether the correction state of each of the left and right subject eyes EL and ER is in a complete correction state (proper) (whether the correction is not overcorrection or undercorrection) using a characteristic of light referred to as chromatic aberration using the RG chart. In addition, in the "RG chart", for example, red icons having a number with a different size and a double circle and a circle with a different size as visual targets and green icons with visual targets having the same shape as the red icons are arranged side by side. In the RG test, due to the characteristics of the light transmissive body of the eye, as the wavelength of light is shorter, the power on the refractive surface is larger. Therefore, the green wavelength light forms an image at a position shifted in the incident side direction as compared with the red wavelength light. Therefore, when the left and right subject eyes EL and ER are undercorrected, the red visual target is more clearly viewed, and when the left and right subject eyes EL and ER are overcorrected, the green visual target is more clearly viewed. Therefore, step S4 and step S5 are repeated until the green and red visual targets of the RG chart are equally viewed, and the subject adjusts the spherical power (correction degree) of the left and right subject eyes EL and ER by changing the position of the focusing lens 45. As a result, the left and right subject eyes EL and ER are prescribed by the complete correction value based on a provisional determination value in the RG test.

Note that steps S1 to S4 correspond to a complete correction prescription step of bringing the left and right subject eyes EL and ER into a state where a complete correction value in the subjective examination is prescribed. However, the complete correction values of the left and right subject eyes EL and ER may not be provisional determination values in the RG test in Example 1, but may be any value of, for example, a value separately measured, a prescription value in the current eyeglasses, a value determined by performing the subjective optometry in a general way, for example. In addition, at this time, the eye positions of the subject eyes EL and ER may be corrected by an examination for heterophoria or strabismus.

Step S5 is a visual target presentation step in which the subjective examination processing portion 144 sets an initial parallax angle and the examination distance to match a convergence distance to a convergence position when the visual target for the left eye and the visual target for the right eye are fused, with the examination distances from the left and right subject eyes EL and ER to the visual target positions. For example, the subjective examination processing portion 144 controls the parallax angle, which sets the initial parallax angle to 0° where the no parallax condition is satisfied, and controls the focusing lens to set the examination distance from the left and right subject eyes, EL and ER at 1 m. This step S5 corresponds to the visual target presentation step of presenting the visual target for the left eye and the visual target for the right eye having the same drawing pattern in the same position where the positions in the up and down direction to the visual target frame on the display 41 (visual target presentation portion) are the same to the visual target position separated from the left and right subject eyes EL and ER by a certain examination distance, subsequent to the prescription of the complete correction value.

Step S6 is a subjective response step in which the subjective examination processing portion 144 acquires a subjective response regarding fusion of the visual target for the left eye and the visual target for the right eye from the subject. The subjective examination processing portion 144, as the subjective response, acquires, for example, a response as to whether the visual targets are seen as one visual target by fusion of the visual target for the left eye and the visual target for the right eye. Here, when the visual targets are not seen as one visual target, it is checked whether the subject eye can be appropriately examined. Then, when the eye cannot be examined, setting is performed again so as to be in an appropriate state, and when the eye can be examined, examination may be stopped, or examination may be continued with eye positions corrected so that the visual targets are seen as one visual target.

Step S7 is a parallax angle change processing step in which the subjective examination processing portion 144 changes the parallax angle in a stepwise manner from the initial parallax angle 0° to the next parallax angle with the examination distance (for example, 1 m) maintained. For example, the parallax angle is switched to the first parallax angle +0.5° in the approaching direction from the initial parallax angle 0° to be changed from the first parallax angle 0.5° to the final parallax angle +0.5°, is switched to the first parallax angle - 0.5° in the separating direction after returning to the initial parallax angle 0°, and is changed in 11 steps (0°, ±0.5°, ± 1°, ±2°, ±3°, ±5°) of from the first parallax angle - 0.5° to the final intersected parallax angle -5°. Note that, this example examines in the direction approaching the subject eye from the parallax angle 0°, returns to the parallax angle 0° after changing to a predetermined parallax angle, and examines in the direction separating from there. However, the present disclosure is not limited thereto, and this example may examine in the approaching direction after the examining in the separating direction first. In addition, when the approaching direction and the separating direction are switched, the subjective examination processing portion 144 may continue the examination after examining again when the parallax angle is returned to 0°. The subjective examination processing portion 144 indicates "parallax" by the parallax angle (angle) that is the angle difference of the eyeball when both eyes observe the visual target closely. However, "parallax" is indicated by the parallax distance (for example, pixel, mm, and cm) in the right and left direction of the two drawing patterns in the position of the examination distance, in addition to the parallax angle. Here, the parallax angle in the direction that rotates an eye inwardly is positive, and the parallax angle in the direction that rotates an eye outwardly is negative.

Step S8 is a subjective response step in which the subjective examination processing portion 144 acquires a subjective response related to fusion of the visual target for the left eye and the visual target for the right eye from the subject, when the fusion is attempted with the left and right subject eyes EL and ER with respect to the stepwise change in the convergence distance. The subjective examination processing portion 144, as the subjective response, acquires, for example, a response as to whether the visual targets are seen as one visual target by fusion is acquired.

Step S9 is a final parallax angle determination step in which the subjective examination processing portion 144 determines whether the parallax angle is changed to the final parallax angle (for example, -5°). While the subjective examination processing portion 144 determines NO in step S9, the process returns to step S7, and when the subjective examination processing portion 144 determines YES in step S9, the process proceeds to step S13.

Steps S7 and S9 correspond to the convergence distance control step of prescribing a state in which the convergence distance from the positions of the left and right subject eyes EL and ER to the convergence position where the two lines of sight intersect each other changes by control that changes a parallax angle from the left and right subject eyes EL and ER toward the visual target for the left eye and the visual target for the right eye. Steps S6 and S8 correspond to a subjective response acquisition step of acquiring a subjective response related to fusion from the subject when fusion is attempted by the left and right subject eyes EL and ER with respect to a change in the convergence distance.

Step S10 is a refractometry step in which the objective measurement portion 145 measures the eye characteristics of the left and right subject eyes EL and ER with respect to the change in the convergence distance with the objective measurement optical system and acquires the objective refraction value as the objective measurement information. Step S11 is a refractometry value display step in which the objective measurement portion 145 displays a plurality of objective refraction values (refractometry values) acquired at the timing when the parallax angle changes. Step S12 is an average refractometry value calculation step in which the objective measurement portion 145 compares a plurality of refractometry values acquired at the timing when the parallax angle changes and calculates an average value. These steps S10 to S12 correspond to the accommodation reaction amount measurement step of being executed in parallel with the control of the convergence distance, measuring the eye characteristics of the left and right subject eyes EL and ER with the objective measurement optical system, when fusion is attempted by the left and right subject eyes EL and ER to the change of the convergence distance, and acquiring the objective refraction value as the objective measurement information.

Step S13 is a step in which the determination portion 146 determines adaptation abilities of the left and right subject eyes EL and ER to the stereoscopic vision based on the subjective response from the subjective examination processing portion 144 and the objective refraction value (average refractometry value) from the objective measurement portion 145. The process proceeds to the end, when the determination of the adaptation abilities of the stereoscopic vision is completed in step S13. This step S13 corresponds to a determination step of determining the adaptation abilities of the left and right subject eyes EL and ER to the stereoscopic vision based on the subjective response and the objective refraction value.

The flow of actions of each step is described as follows. The process of FIG. 4 starts when alignment in which the subject faces the ophthalmic examination apparatus 100 and sets the face at a predetermined position, and appropriately adjusts the position relationship between the left and right subject eyes EL and ER and the apparatus is completed, and the process proceeds from step S1 to step S2 to step S3 and to step S4. After starting, the main controller 141 performs preliminary measurement (step S1), main measurement (step S2), and start setting of subjective examination (step S3). In step S4, after the start setting of the subjective examination, the main controller 141 performs the RG test using the RG chart, thereby bringing the left and right subject eye EL and ER into a state in which the complete correction value is prescribed by the provisional determination value in the RG test.

The process of FIG. 4 proceeds from step S5 to step S6 after the complete correction value is in a state of being prescribed for the left and right subject eyes EL and ER in step S4. In step S5, the subjective examination processing portion 144 performs visual target switching control in the main controller to set the parallax angle to the initial parallax angle 0° and movement control of the focusing lens 45 to set the examination distance to a constant distance. In step S6, the subjective examination processing portion 144 acquires, from the subject, a subjective response related to fusion when the convergence distance and the examination distance are matched.

The process in FIG. 4 proceeds from step S7 to step S8 to step S9 after the subjective examination processing portion 144 acquires the subjective response related to the fusion in the initial setting from the subject. In step S7, the subjective examination processing portion 144 performs the visual target switching control in the main controller 141 that switches the parallax angle while maintaining the switching time and the parallax angle maintaining time from the initial parallax angle 0° in which the convergence distance becomes a convergence position to the next parallax angle (for example, +0.5°). In step S8, the subjective examination processing portion 144 acquires, from the subject, the subjective response related to the fusion when the parallax angle is switched. In step S9, the subjective examination processing portion 144 determines whether the parallax angle is changed to the final parallax angle (for example, -5°) that the convergence distance is farthest from the right and left subject eyes EL and ER.

In the process of FIG. 4, while the subjective examination processing portion 144 determines that the parallax angle is not changed to the final parallax angle (for example, -5°) in step S9, the flow from step S7 to step S8 to step S9 is repeated. In step S7, the subjective examination processing portion 144 switches the parallax angle from the present parallax angle to the next parallax angle by the visual target switching control in the main controller 141. The parallax angle is switched in a stepwise manner from 0° (no parallax) to parallax angle +0.5° to parallax angle +1° to parallax angle +2° to parallax angle +3° to parallax angle +5° to parallax angle -0.5° to parallax angle -1° to parallax angle -2° to parallax angle -3° to parallax angle -5°, and the convergence distance from the left and right subject eyes EL and ER to the virtual position is changed in a stepwise manner. In step S8, the subjective examination processing portion 144 acquires, from the subject, the subjective response related to the fusion in each step of the switched parallax angle.

In the process of FIG. 4, in step S5, when the subjective examination processing portion 144 controls the matching of the convergence distance with the examination distance, the objective measurement portion 145 starts the objective measurement in parallel with the subjective examination process. For the objective measurement, the subjective examination process repeats the flow proceeding to step S10 to step S11 to step S12. When the subjective examination process ends (YES in step S9), the objective measurement ends in accordance with the end of the subjective examination process. In step S10, the objective measurement portion 145 acquires the objective refraction value (refractometry value) as the objective measurement information by the refractometry. n step S 11, the objective measurement portion 145 displays a plurality of objective refraction values (refractometry values) acquired at the timing when the parallax angle changes. In step S12, the objective measurement portion 145 compares a plurality of refractometry values acquired at the timing when the parallax angle changes to calculate the average value.

In the process of FIG. 4, while the subjective examination processing portion 144 determines that the parallax angle is changed to the final parallax angle (for example, -5°) in step S9, the process proceeds from step S9 to step S13. In step S13, the determination portion 146 determines adaptation abilities of the left and right subject eyes EL and ER to the stereoscopic vision based on the subjective response from the subjective examination processing portion 144 and the objective refraction values (average refractometry values) from the objective measurement portion 145.

As described above, the flow of the processing action of evaluating the adaptation abilities to the stereoscopic vision is executed in parallel with the subjective examination process and the objective measurement and merges with the objective measurement in step S13 when the subjective examination process ends. In step S13, for example, when a subjective response that the fusion by binocular vision is achieved is obtained irrelevant to the stepwise change of the convergence distance and the change of the average refractometry value is suppressed in a predetermined range, the determination portion 146 determines that the left and right subject eyes EL and ER have high adaptation abilities to stereoscopic vision. In step S13, for example, by changing the convergence distance in a stepwise manner, when the subjective response that two visual targets are seen is obtained and fusion by binocular vision is not formed, or the change of the average refractometry value exceeds the predetermined range, the determination portion determines 146 determines that the left and right subject eyes EL and ER have low adaptation abilities to stereoscopic vision.

[Visual Target for Left Eye and Visual Target for Right Eye (FIGS. 5A and 5B)] FIGS. 5A and 5B describe a visual target for the left eye 8L and a visual target for the right eye 8R set on the display 41 (visual target presentation portion).

The visual target for the left eye 8L is a visual target projected on the left subject eye EL when the adaptation check portion 143 evaluates the adaptation abilities of stereoscopic vision. The visual target for the right eye 8R is a visual target projected on the right subject eye ER when the adaptation check portion 143 evaluates the adaptation abilities of stereoscopic vision. The visual target for the left eye 8L and the visual target for the right eye 8R share a square-shaped visual target frames 81L and 81R according to a liquid crystal frame, for example, and asterisk marks 82L and 82R (drawing pattern) with the same drawing pattern, same size, and same line width inside the visual target frames 81L and 81R. A pattern, a size, and a line segment width of the asterisk marks 82L and 82R are determined to appropriately provide fusion stimulation to the left and right subject eyes EL and ER at the time of fusion in which images reflected on the retina at the time of binocular vision are combined into one and viewed as a single image.

As shown in FIG. 5A, the two asterisk marks 82L (0°) and 82R (0°) of a pair of the visual mark for the left eye 8L and the visual mark for the right eye 8R of the initial parallax angle are set at the center positions of the insides of the visual target frames 81L, 81R in the left and right direction. Therefore, when the fusion is attempted by the left and right subject eyes EL, ER, the two asterisk marks 82L (0°) and 82R (0°) establish no parallax condition with the visual target positions distant from the left and right subject eyes EL, ER by the examination distance as the fusion positions.

As shown in FIG. 5B, a pair of the visual targets 8L and 8R for the left and right eyes with a parallax angle where the convergence distance is greater than the examination distance can induce the stereoscopic parallax by shifting the position of the two asterisk marks 82L (-5°) and 82R (-5°) respectively, outward to the left or right directions from the center of the visual target frame 81L and 81R. The asterisk mark 82L (-5°) is set to the outside (left side) from the center position in the left and right direction in the visual target frame 81L. The asterisk mark 82R (-5°) is set at a position outside (right side) from the center position in the left and right direction in the visual target frame 81R. Therefore, the two asterisk marks 82L (-5°) and 82R (-5°) are fused in the back fusion positions when the fusion is attempted without intersecting the lines of sight from the left and right subject eyes EL and ER.

As shown in FIG. 5B, a pair of the visual targets 8L and 8R for the left and right eyes can show the asterisk marks 82L and 82R at the positions that correspond to each parallax angle of -0.5°, -1°, -2°, and -3°, in addition to the parallax angle of -5°. Each asterisk mark 82L, 82R is set in the predetermined positions, which are arranged stepwise within the range from the dash-lined asterisk marks 82L (-0.5°) and 82R (-0.5°) to the solid-lined asterisk marks 82L (-5°) and 82R (-5°) in FIG. 5B. Therefore, each asterisk mark 82L, 82R is fused in the fusion position where the amount of withdrawn from the parallax position stepwisely increases from the parallax angle of -0.5° to the parallax angle of -5° when the fusion is attempted by intersecting the lines of sight from the left and right subject eyes EL, ER.

As shown in FIG. 5C, a pair of the visual target for the left eye 8L and the visual target for the right eye 8R with a parallax angle where the convergence distance is shorter than the examination distance can induce the stereoscopic parallax by shifting the positions of the two asterisk marks 82L (+5°) and 82R (+5°) to the visual target frames 81L and 81R respectively, inward to the left or right directions from the center of the visual target frame 81L and 81R. The asterisk mark 82L (+5°) is set inward (right side) from the center position in the left and right direction in the visual target frame 81L. The asterisk mark 82R (+5°) is set inward (left side) from the center position in the left and right direction in the visual target frame 81R. Therefore, the two asterisk marks 82L (+5°) and 82R (+5°) have the fusion positions protruding from the visual target positions when the fusion is attempted by intersecting the lines of sight from the left and right subject eyes EL and ER.

As shown in FIG. 5C, a pair of the visual target for the left eye 8L and the visual target for the right eye 8R can show the asterisk marks 82L and 82R at the positions that correspond to each of the parallax angles of +0.5°, +1°, +2°, and +3°, in addition to the parallax angle of +5°. Each asterisk mark 82L, 82R is set in the predetermined positions which are arranged stepwise within the range from the dashed asterisk marks 82L (+0.5°) and 82R (+0.5°) in FIG. 5C to the solid asterisk marks 82L (+5°) and 82R (+5°) in FIG. 5C. Therefore, each asterisk mark 82L, 82R is fused in the fusion position where the protruding amount increases in a stepwise manner from the parallax angle +0.5° toward the parallax angle +5° when the fusion is attempted by intersecting the lines of sight from the left and right subject eyes EL, ER.

Since the liquid crystal frames used in the visual target frames 81L and 81R can induce weak fusion stimulation y, the visual target frames 81L and 81R employ a black frame with a thicker line width, which can increase the fusion stimulation. Furthermore, the visual target frames 81L and 81R can also have variable strength of (the) fusion stimulation by changing the line width of the black frames. The asterisk marks 82L and 82R can also have variable fusion stimulation strength by changing the size and radial-line width. Furthermore, the asterisk marks 82L and 82R can also have variable fusion stimulation strength by changing the drawing pattern of the visual target.

[Relationship Between Examination Distance and Convergence Distance (FIG. 6)] The subjective examination processing portion 144 prescribes a state in which the convergence distance from the positions of the left and right subject eyes EL and ER to the convergence position where the two lines of sight intersect changes by the control that changes a parallax angle from the left and right subject eyes EL and ER toward the visual target for the left eye 8L and the visual target for the right eye 8R. FIG. 6 describes the relationship between the convergence distance and the examination distance.

As illustrated in FIG. 6, the term "examination distance Lid" refers to a distance from the left and right subj ect eyes EL and ER to a position where the visual target is presented in the Z-axis direction. The examination distance Lid is obtained by the visual target projection system 4 that presents the visual target on the display 41. In addition, the examination distance Lid can be referred to as a focus accommodation distance when the visual target is focused by the left and right subject eyes EL and ER. Here, the examination distance Lid is the distance of the visual target from the straight line connecting the left and right subject eyes EL and ER, but is not limited thereto, and may be the distance of the visual target from each of the subject eyes EL and ER.

The convergence distance L3 is the distance along the Z-axis direction from the position of the left and right subject eyes EL and ER to the convergence position R3 where the two lines of sight SL3, SR3 intersect when the two asterisk marks 82L and 82R are gazed without intersecting the two lines of sight SL3 and SR3 from the left and right subject eyes EL and ER, as shown in FIG. 6. Therefore, the convergence distance L3 is longer than the examination distance Lid to the visual target position where a pair of the visual target for the left eye 8L and the visual target for the right eye 8R is presented.

As shown in FIG. 6, the convergence distance L2 is the distance along the Z-axis direction from the position of the left and right subject eyes EL and ER to the convergence position R2 where the two lines of sight SL2 and SR2 intersect, when the two lines of sight SL2 and SR2 from the left and right subject eyes EL and ER intersect at the visual target position. Therefore, the convergence distance L2 corresponds to the examination distance Lid to the visual target position where a pair of the visual target for the left eye 8L and the visual target for the right eye 8R is presented.

As shown in FIG. 6, the convergence distance L1 is the distance along the Z-axis direction from the position of the left and right subject eyes EL and ER to the convergence position R1 where the two lines of sight SL1 and SR1 intersect when the two asterisk marks 82L and 82R are gazed by intersecting the two lines of sight SL1 and S1 from the left and right subject eyes EL and ER. Therefore, the convergence distance L2 is shorter than the examination distance Lid to the visual target position where a pair of the visual target for the left eye and the visual target for the right eye 8L and 8R is presented.

Here, the two asterisk marks 82L and 82R are arranged in positions separated by the parallax distance. The parallax angle at the convergence position R3 obtained without intersecting the two lines of sight SL3 and SR3 is calculated by the parallax angle (R3) = (parallax angle Θ3 - parallax angle θ2), which is a negative value and means the separating direction. The parallax angle at the convergence position R1 obtained by intersecting the two lines of sight SL1 and SR1 is calculated by the parallax angle (R1) = (parallax angle θ1 - parallax angle θ2), which is a positive value and means the approaching direction.

While the examination distance is kept at the examination distance Lid, the convergence distance is changed from, for example, the convergence distance L2 to the convergence distance L3. The convergence angle changes from the convergence angle θ2 where the lines of sight SL2 and SR2 intersect to the convergence angle θ3 (<Θ2) where the lines of sight SL3 and SR3 intersect. Therefore, as the convergence angle changes from θ2 to θ3, the eye movement of the left and right subject eyes EL and ER occurs in the direction where the left and right subject eyes EL and ER are separated, and this eye movement eases the convergence.

While the examination distance is kept at the examination distance Lid, the convergence distance is changed from, for example, the convergence distance L2 to the convergence distance L1. The convergence angle changes from the convergence angle θ2 where the lines of sight SL2 and SR2 intersect to the convergence angle θ1 (>θ2) where the lines of sight SL3 and SR3 intersect. Therefore, as the convergence angle changes from θ2 to θ1, the eye movement of the left and right subject eyes EL and ER occurs in the direction where the left and right subject eyes EL and ER approach each other, and this eye movement stimulates the convergence.

Meanwhile, since the examination distance Lid is maintained at a certain distance, the focus accommodation distance by the left and right subject eyes EL and ER is also constant. Therefore, maintaining the focus accommodation distance of the left and right subject eye EL and ER at a certain distance fails to work the accommodation by the change in the examination distance Lid although the convergence distance is changed from the convergence distance L3 to the convergence distance L1. Therefore, no objective refraction value changes in principle when the left and right subject eyes EL and ER are objectively measured. However, when the accommodation that changes the curvature of the lens eye works due to the convergence stimulation, the objective refraction values of the left and right subject eyes EL and ER change when the left and right subject eyes EL and ER are objectively measured.

As described above, the relationship in which the examination distance is kept at a certain distance, and the convergence distance change is a relationship which provides the convergence stimulation only to the left and right subject eyes EL and ER. Therefore, the adaptation check portion 143 of Example 1 has a configuration which reproduces a state in which the convergence stimulation is given by the change in a parallax angle, and discrepancy occurs between the convergence and the accommodation when the two visual targets are arranged at positions at a certain distance from the left and right subject eyes EL and ER, and the two visual targets are fused by both eyes. For this reason, the adaptation check portion 143 can evaluate whether the left and right subject eyes EL and ER have high adaptation abilities to stereoscopic vision in which the discrepancy between the convergence and the accommodation occurs.

[Stepwise Changing Action of Convergence Distance (FIGS. 7A to 7C)] The subjective examination processing portion 144 prescribes a state in which a convergence position R and a convergence distance L change in a stepwise manner in a distance range from a position farther than the examination distance Lid to a position closer than the examination distance Lid by that control that changes the parallax angle in a stepwise manner. FIGS. 7A to 7C describe the stepwise changing action of the convergence distance L.

FIG. 7A illustrates a state in which the convergence distance L (0°) to the convergence position R (0°) when the two asterisk marks 82L (0°) and 82R (0°) are fused is matched with the examination distance Lid from the left and right subject eyes EL and ER to the visual target position (step S5). FIG. 7B shows a state in which the convergence distance L (+5°) to the convergence position R (+5°) when the two asterisk marks 82L (0°) and 82R (0°) are fused by intersecting the lines of sight is shorter than the examination distance Lid from the left and right subject eyes EL and ER to the visual target position. The convergence distance L has the relationship L (+0.5°) > L (+1°) > L (+2°) > L (+3°) > L (+5°), where the larger the parallax angle, the shorter the parallax distance. FIG. 7C shows a state in which the convergence distance L (-5°) to the convergence position R (-5°) when the two asterisk marks 82L (-5°) and 82R (-5°) are fused without intersecting the lines of sight is longer than the examination distance Lid from the left and right subject eyes EL and ER to the visual target position. The convergence distance L has the relationship L (-0.5°) <L (-1°) <L (-2°) <L (-3°) <L (-5°), where the larger the parallax angle, the longer the parallax distance.

In step S5 of FIG. 4, the subjective examination processing portion 144 sets the examination distance Lid to 1 m and sets the parallax angle to the initial parallax angle (0°). Therefore, when the initial parallax angle (0°) is selected, the convergence position R (0°) when the two asterisk marks 82L (0°) and 82R (0°) are fused is aligned with the visual target position, as illustrated in FIG. 7A.

In step S7 of FIG. 4, the subjective examination processing portion 144 changes the parallax angle in 11 stages of 0°, ±0.5°, ±1°, ±2°, ±3°, and ±5° while maintaining the examination distance Lid (for example, 1 m). As illustrated in FIG. 7C, when the parallax angle of -0.5° is selected, the convergence position R (-0.5°) when the two asterisk marks 82L (-0.5°) and 82R (-0.5°) are fused is farther than the examination distance Lid. When the parallax angle is changed in five steps from -0.5° to -1°, -2°, -3°, and -5°, the convergence position R separates in a stepwise manner from the position of the left and right subject eyes EL and ER in the range farther than the examination distance Lid. When the parallax angle of -5° is selected, the convergence position R (-5°) when the two asterisk marks 82L (-5°) and 82R (-5°) are fused become the position farthest from the left and right subject eyes EL and ER, as shown in FIG. 7C.

When a parallax angle of +0.5° is selected, the convergence position R (+0.5°) when the two asterisk marks 82L (+0.5°) and 82R (+0.5°) are fused is closer than the examination distance Lid. When the intersected parallax angle is changed in five steps from 0.5°, +1°, +2°, +3°, and +5°, the convergence position R approaches the left and right subject eyes EL and ER in a stepwise manner in the region closer than the examination distance Lid. When the parallax angle of +5° is selected, the convergence position R (+5°) when the two asterisk marks 82L (+5°) and 82R (+5°) are fused is closest to the left and right subject eyes EL and ER, as shown in FIG. 7B.

Thus, when the subjective examination processing portion 144 changes the parallax angle in a stepwise manner, the subjective examination processing portion 144 changes the convergence distance L (±n°) from the state that the convergence distance approaches from the examination distance Lid to the state that the convergence distance separates from the examination distance Lid in a stepwise manner. Therefore, by changing the convergence distance L (±n°), the subjective examination processing portion 144 can provide convergence stimulation that changes stepwise to the left and right subject eyes EL and ER.

For the fusion by the parallel method, which does not intersect the lines of sight, a brain perceives that the two asterisk marks 82L (-5°) and 82R (-5°) of the visual target for the left eye 8L and the visual target for the right eye 8R are superimposed on the retinas of the left and right subject eyes EL and ER, respectively, as shown in FIG. 7C. Therefore, portions of the two visual target frames 81L and 81R overlap each other in a misaligned position relationship, and have at the center the asterisk marks 82L (-5°) and 82R (-5°), which are superimposed by the fusion. Therefore, the asterisk marks 82L (-5°) and 82R (-5°), which are fused at a position farther from the visual target position, can appear stayed backward to the visual target frames 81L and 81R in the overlapped portion of the two frames.

Similarly, for the fusion by the intersecting method that intersects the lines of sight, the brain perceives that the two asterisk marks 82L (+5°), 82R (+5°) of the visual target for the left eye 8L and visual target for the right eye 8R are superimposed on the retinas of the left and right subject eyes EL and ER, respectively, as shown in FIG. 7B. Therefore, the portions of the two visual target frames 81L and 81R overlap each other in a misaligned position relationship, and have at the center the asterisk marks 82L (+5°) and 82R (+5°), which are superimposed by the fusion. Therefore, the asterisk marks 82L (+5°) and 82R (+5°), which are fused closer to the visual target position, can appear to protrude to the visual target frames 81L and 81R in the overlapped portion of the two frames.

[Measurement of Temporal Change of Accommodation and Convergence during Stereoscopic Image Observation (FIGS. 8A to 8E)] The present inventors measured the temporal change of the accommodation and the convergence separately for subject eyes of a healthy group and subject eyes of a stereoscopic image fatigue group. Hereinafter, FIGS. 8A to 8E describe the measurement of the temporal change of the accommodation and convergence.

As shown in FIG.8A, the stereoscopic image is observed by using the naked-eye stereoscopic display which include the real visual target, that appears three-dimensional to naked eyes. The naked-eye stereoscopic display has a viewing distance of 60 cm from the left and right subject eyes, a parallax distance of 4 cm (parallax angle of 3.8°), and a stereoscopic image protruding amount of 24 cm. As shown in FIG. 8B, for the measurement conditions, the parallax angle of 0° is maintained from 1 to 2 seconds, the intersected parallax angle is changed from 0° to 3.8° over 2 seconds, and then the intersected parallax of 3.8° is fixed.

FIG. 8B shows the measurement results by the healthy eye example. When the intersected parallax angle is changed from 0° to 3.8° over 2 seconds, the value of the convergence (MA) decreases following the change in the intersected parallax angle. When the intersected parallax angle of 3.8° is fixed, the value of the convergence (MA) slightly fluctuates, but the value due to the protruding position after the decrease is maintained. Note that the term "Meter Angle (MA)" is a unit of convergence force. When the intersected parallax angle is changed from 0° to 3.8° over 2 seconds, the focus accommodation function for the protruding position works to decrease the value of the refractivity (D). However, when the intersected parallax angle of 3.8° is fixed, the decreased value of the refractivity (D) returns to the original value with a good response, and then the original value is maintained. Note that the term "Diopter (D)" is a unit of the refractivity in which the refractive power is replaced with a number.

FIG. 8D shows the measurement results of the stereoscopic image fatigue group ar. The measurement results of the convergence (MA) and the refractivity (D) in FIG. 8D (a) are an example in which a user feels a headache and eye fatigue at the time of viewing an IMAX movie ("IMAX" is a registered trademark) for about 15 minutes and experiences discontinuation of viewing. Here, the "IMAX movie" refers to a movie based on a high-definition video shown with a special projection system using a moving image film of a special standard (a standard in which the area of a film used for one frame is widened from a normal 35 mm to 70 mm). Irrelevant to the change or maintenance of the intersected parallax angle, both the convergence (MA) and the refractivity (D) show disturbed characteristics such as including fluctuation of a large value and interruption of a value in the middle.

The measurement results of the convergence (MA) and the refractivity (D) in FIG. 8D (b) are an example in which a user feels strong sleepiness and eye fatigue at the time of viewing a 3D movie for about 30 minutes and experiences discontinuation of viewing. Here, the "3D movie" refers to a movie in which a video projected on a screen is stereoscopically shown with special glasses or the like. When the intersected parallax angle is changed from 0° to 3.8° over 2 seconds, the value of the convergence (MA) at the time of start of the change is lower than that in case of the healthy eye example but decreases following the change in the parallax angle. When the intersected parallax angle of 3.8° is fixed, the value of the convergence (MA) depending on the protruding position tends to be maintained, but the value is interrupted in the middle, which indicates a disturbed characteristic. When the intersected parallax angle is changed from 0° to 3.8° over 2 seconds, the focus accommodation function for the protruding position works to decrease the value of the refractivity (D). When the intersected parallax angle of 3.8° is fixed, the refractivity (D) shows a disturbed characteristic due to the fluctuation of a value for returning a decreased value to an original value or a value interruption in the middle.

FIG. 8E shows the analysis result examples of the temporal change frequency of the refractivity (D) by the healthy group and the fatigue group. When the magnitude of the amplitude is compared between the healthy group and the fatigue group, the amplitude (fluctuation range) is larger in the fatigue group than in the healthy group in any of the frequency bands of 0.5 to 1.5 Hz, 1.5 to 2.5 Hz, 2.5 to 3.5 Hz, and 3.5 to 4.5 Hz. Therefore, the analysis result example illustrated in FIG. 8E can be used as data for checking that the subject eye belongs to the fatigue group with the magnitude of the fluctuation range when the amplitude (fluctuation range) of the temporal change frequency of the refractivity (D) of the subject eye is measured. In particular, in a low frequency band, the analysis result example can be used as the data for checking that the subject eye belongs to the fatigue group with the magnitude of the amplitude.

As illustrated in FIG. 8E, for example, in a low frequency band of 0.5 to 1.5 Hz, the determination threshold for determining that the subject eye belongs to the fatigue group is set to about an amplitude of 0.2 to 0.3. In this case, when the stereoscopic adaptation evaluation examination is performed on a predetermined subject eye, and the objective refraction value data is acquired in time series along the time axis, the amplitude in the low frequency band of 0.5 to 1.5 Hz of the objective refraction value characteristic by the data is measured. Then, when the measured amplitude of the low frequency band is a preset determination threshold or more, the subject eye can be determined as belonging to the fatigue group. Note that, here, the examination is performed by changing the convergence distance with the naked-eye stereoscopic display, but the present disclosure is not limited thereto. For example, a similar examination can be performed by presenting an image in which the line of sight angle is changed to each of left and right eyes by changing an image that is projected on the left and right eyes with an internal visual target.
a naked-eye stereoscopic display and presenting an image in which the line-of-sight angle is changed to each of the left and right subject eyes.

[Action of determining adaptation ability to Stereoscopic Vision (FIG. 9)] The objective measurement portion 145 measures the eye characteristics of the left and right subject eyes EL and ER a predetermined number of times in each step of the convergence distance L and acquires an average objective refraction value obtained by averaging the objective refraction values obtained a predetermined number of times in each step as the objective measurement information. FIG. 9 describes the action of determining the adaptation ability to stereoscopic vision.

FIG. 9 illustrates a relationship characteristic between the parallax angle and the objective refraction value when the average objective refraction value is acquired in each step of the convergence distance L for a plurality of subjects. The vertical axis represents an objective refraction value, and the unit of each numerical value is a diopter (D). The horizontal axis represents a parallax angle, and the unit of each numerical value is a degree (°) but may be converted into a diopter (D) or a convergence angle (MA) represented by a reciprocal number of a convergence distance.

A first group G1 of the relationship characteristics in FIG. 9 shows a characteristic that the numerical value of the objective refraction value tends to rise in response to the switching of the parallax angle when the parallax angle is changed in seven steps of -0.5° to 0° to 0.5° to +1° to +2° to +3° to +5°. That is, when the intersected parallax angle becomes - 0.5° to +1° to +2° to +3° to +5°, the first group G1 has a characteristic that the numerical value of the objective refraction value rises to about D = 3.0 accordingly.

For this reason, the focus accommodation reactions of the left and right subject eyes EL and ER of the subject belonging to the first group G1 may be induced, while the subject belonging to the first group G1 receives convergence stimulation due to a change in the convergence distance. Therefore, the discrepancy between the refraction value and the visual target position occurs due to the convergence, and thus the left and right subject eyes EL and ER of the subject belonging to the first group G1 can be determined as having low adaptation abilities to stereoscopic vision.

Meanwhile, a second group G2 of the relationship characteristics in FIG. 9 shows a characteristic that the numerical value of the objective refraction value transitions in a state of crawling, when the parallax angle is changed in seven steps of -0.5° to 0° to 0.5° to +1° to +2° to +3° to +5°. That is, even when the parallax angle becomes -0.5° to 0° to +0.5° to +1° to +2° to +3° to +5°, the second group G2 has a characteristic that the numerical value of the objective refraction value is suppressed to about D < 1.0.

For this reason, the focus accommodation reactions of the left and right subject eyes EL and ER of the subject belonging to the second group G2 may not be induced, even when the subject belonging to the second group G2 receives convergence stimulation due to a change in the convergence distance. Therefore, the discrepancy between the refraction value and the visual target position does not occur due to the convergence, and thus the left and right subject eyes EL and ER of the subject belonging to the second group G2 can be determined as having high adaptation abilities to stereoscopic vision.

[Effects of Ophthalmic Examination Apparatus and Ophthalmic Examination Method] (1) The ophthalmic examination apparatus 100 includes the visual target projection system 4 including the visual target presentation portion (display 41) that presents fixed visual targets to the left and right subject eyes EL and ER, the objective measurement optical systems 6 and 7 that objectively measure the eye characteristics of the left and right subject eyes EL and ER, and the controller 140 that controls each portion of the apparatus. The fixed visual targets are the visual target for the left eye 8L and the visual target for the right eye 8R which are projected onto the left and right subject eyes EL and ER, respectively, and have the same drawing pattern (asterisk mark 82L and 82R) in the positions where the up and down direction positions to the visual target frames 81 are the same. The visual target presentation portion presents the left eye projection visual target 8L and the right eye projection visual target 8R at the visual target positions separated from the left and right subject eyes EL and ER by the certain examination distance Lid. The visual target projection system 4 prescribes a state in which the convergence distance L from the positions of the left and right subject eyes EL and ER to the convergence position R where the two lines of sight intersect each other changes by changing a parallax angle that is a difference in the positions of the two drawing patterns (asterisk marks 82L and 82R) reflected on the retinas of the left and right subject eyes EL and ER. When fusion is attempted with the left and right subject eyes EL and ER to a change in the convergence distance L, the controller 140 measures the eye characteristics of the left and right subject eyes EL and ER with the objective measurement optical systems to acquire an objective refraction value as the objective measurement information. For this reason, the ophthalmic examination apparatus 100 can evaluate whether the left and right subject eyes EL and ER are easily adapted to stereoscopic vision in which discrepancy between convergence and accommodation occurs.

(2) The ophthalmic examination apparatus 100 is an objective measurement machine with a subjective examination function. The controller 140 includes the adaptation check portion 143 that evaluates the adaptation abilities by the left and right subject eyes EL and ER, and the adaptation check portion 143 includes the subjective examination processing portion 144 and the objective measurement portion 145. The subjective examination processing portion 144 presents the visual target for the left eye 8L and the visual target for the right eye 8R at the visual target positions separated from the left and right subject eyes EL and ER by the certain examination distance Lid. A state in which the convergence distance L from the positions of the left and right subject eyes EL and ER to the convergence position R where the two lines of sight intersect each other changes is prescribed by the control that changes the parallax (parallax angel) that is a difference in the positions of the two drawing patterns (asterisk marks 82L and 82R) reflected on the retinas of the left and right subject eyes EL and ER. The objective measurement portion 145 executes processes in parallel with the process in the subjective examination processing portion 144, measures the eye characteristics of the left and right subject eyes EL and ER with the objective measurement optical systems 6 and 7 when fusion is attempted with the left and right subject eyes EL and ER to a change in the convergence distance L, and acquires an objective refraction value as the objective measurement information. Therefore, the ophthalmic examination apparatus 100 can save an effort and time required for the subjective examination as compared with the case where the subjective examination process is performed by a manual operation. In addition, the ophthalmic examination apparatus 100 can accurately evaluate whether the left and right subject eyes EL and ER have high adaptation abilities to stereoscopic vision by executing the subjective examination process and the objective measurement by concurrent processing by simultaneous progress.

(3) The visual target for the left eye 8L and the visual target for the right eye 8R are stereoscopic visual targets provided with parallax by differentiating the positions of the displayed drawing patterns (asterisk marks 82L and 82R) in the left and right direction to differentiate the left and right positions of the two drawing patterns (asterisk marks 82L and 82R) to the visual target frames 81L and 81R. The subjective examination processing portion 144 performs the control that changes the parallax angle as the control that changes the parallax angle or the parallax distance when the fusion of the two drawing patterns (asterisk marks 82L and 82R) is attempted by binocular vision. Therefore, when the subjective examination processing portion 144 performs the control that changes the parallax angle or the parallax distance, the subjective examination processing portion 144 can set the fused asterisk marks 82L and 82R at the withdrawn position or at the protruding position. In addition, the ophthalmic examination apparatus 100 can measure eye positions and directions of the lines of sight for checking whether objective binocular vision can be performed with the left and right subject eyes EL and ER from the anterior segment image by changing a pair of visual targets while the visual axis and the optical axis are aligned.

(4) When the fusion is attempted with the left and right subject eyes EL and ER with respect to the change in the convergence distance, the subjective examination processing portion 144 acquires a subjective response from the subject. The adaptation check portion 143 includes, in addition to the subjective examination processing portion 144 and the objective measurement portion 145, the determination portion 146 that determines the adaptation abilities of the left and right subject eyes EL and ER to stereoscopic vision based on the subjective response from the subject acquired by the subjective examination processing portion 144 and the objective refraction value acquired by the objective measurement portion 145. Therefore, when the determination portion 146 determines the adaptation abilities of the left and right subject eyes EL and ER to stereoscopic vision, the determination portion 146 can check whether fusion is subjectively achieved.

(5) When a positive subjective response to the fusion by binocular vision is obtained with respect to the change of the convergence distance L, and the change width of the objective refraction value is suppressed in a predetermined range, the determination portion 146 determines that the left and right subject eyes EL and ER have high adaptation abilities to stereoscopic vision. For this reason, when the fusion is subjectively generated while the convergence stimulation is given by the change in the convergence distance L and the focus accommodation reaction of the left and right subject eyes EL and ER is blunt, the determination portion 146 can determine that the left and right subject eyes EL and ER have high adaptation abilities to stereoscopic vision.

(6) When a negative subjective response to the fusion by binocular vision is obtained with respect to the change of the convergence distance L, and the change width of the objective refraction value exceeds a predetermined range, the determination portion 146 determines that the left and right subject eyes EL and ER have low adaptation abilities to stereoscopic vision. For this reason, when the subjective fusion is difficult while the convergence stimulation is given by the change in the convergence distance L, and the focus accommodation reaction of the left and right subject eyes EL and ER occurs, the determination portion 146 can determine that the left and right subject eyes EL and ER have high adaptation abilities to stereoscopic vision.

(7) The adaptation check portion 143 includes, in addition to the subjective examination processing portion 144 and the objective measurement portion 145, the determination portion 146 that determines the adaptation abilities of the left and right subject eyes EL and ER to stereoscopic vision based on the objective refraction value acquired by the objective measurement portion 145. The determination portion 146 measures the amplitude which is the fluctuation range of the refraction value characteristic with the time axis of the objective refraction value acquired by the objective measurement portion 145 and determines that the left and right subject eyes EL and ER belong to the fatigue group when the fluctuation frequency of the refraction value characteristic is in the low frequency band and the amplitude is the determination threshold or more. Therefore, the determination portion 146 can determine that the left and right subject eyes EL and ER belong to the fatigue group that has low adaptation abilities to stereoscopic vision based on the refraction value characteristic with the time axis of the acquired objective refraction value.

(8) The subjective examination processing portion 144 prescribes a state in which the convergence distance L changes in a stepwise manner in a distance range from a position farther than the examination distance Lid to a position closer than the examination distance Lid by control that changes the parallax angles that is the difference in the positions of the two drawing patterns (asterisk marks 82L and 82R) reflected on the retinas of the left and right subject eyes EL and ER. The objective measurement portion 145 measures the eye characteristics of the left and right subject eyes EL and ER a predetermined number of times in each step of the convergence distance L and acquires an average objective refraction value obtained by averaging the objective refraction values obtained a predetermined number of times in each step as the objective measurement information. Therefore, the subjective examination processing portion 144 can give convergence stimulation that changes in a stepwise manner to the left and right subject eyes EL and ER. In addition, the objective measurement portion 145 can acquire information indicating the accommodation reaction amount of the left and right subject eyes EL and ER with respect to a convergence stimulation that changes in a stepwise manner, by an average objective refraction value that is objective measurement information.

(9) The ophthalmic examination method includes the complete correction prescription step (steps S1 to S4), the visual target presentation step (step S5), the convergence distance control step (step S7 and step S9), the accommodation reaction amount measurement step (steps S10 to S12), and a determination step (step S13). The complete correction prescription step assumes the left and right subject eyes EL and ER as a state in which a complete correction value in subjective examination is prescribed. The visual target presentation step presents the visual target for the left eye 8L and the visual target for the right eye 8R having the same drawing pattern (asterisk mark 82L and 82R) in the positions where the up and down direction positions to the visual target frames 81 of the visual target presentation portion (display 41) are the same at the visual target position separated from the left and right subject eyes EL and ER by the certain examination distance Lid, subsequent to the prescription of the complete correction value. The convergence distance control step prescribes a state in which the convergence distance L from the positions of the left and right subject eyes EL and ER to the convergence position R where the two lines of sight intersect each other changes by changing the parallax (parallax angle) that is the difference in the positions of the two drawing patterns (asterisk marks 82L and 82R) reflected on the retinas of the left and right subject eyes EL and ER. The accommodation reaction amount measurement step is executed in parallel with the control of the convergence distance L, measures the eye characteristics of the left and right subject eyes EL and ER with the objective measurement optical systems 6 and 7 when fusion is attempted by the left and right subject eyes EL and ER to the change of the convergence distance L, and acquires the objective refraction value as the objective measurement information. The determination step determines adaptation abilities of the left and right subject eyes EL and ER to stereoscopic vision based on the objective refraction value. For this reason, the ophthalmic examination method (FIG. 4) can evaluate whether the left and right subject eyes EL and ER have high adaptation abilities to stereoscopic vision in which discrepancy between convergence and accommodation occurs. In addition, the ophthalmic examination method (FIG. 4) can perform training for improving the adaptation abilities to stereoscopic vision by utilizing the convergence distance control step and causing the left and right subject eyes EL and ER to repeatedly observe and habituate those having the convergence distances L shifted.

(10) The ophthalmic examination method includes the subjective response acquisition step (step S6, step S8) of acquiring a subjective response related to fusion from the subject when fusion is attempted by the left and right subject eyes EL and ER with respect to a change in the convergence distance. The determination step determines adaptation abilities of the left and right subject eyes EL and ER to stereoscopic vision based on the subjective response and the objective refraction value. Therefore, when determining the adaptation abilities of the left and right subject eyes EL and ER to stereoscopic vision, the ophthalmic examination method (FIG. 4) can check whether fusion is subjectively achieved.

### Example 2

Example 2 is an example of prescribing a state in which the convergence distance L changes smoothly in a distance range from a position farther than the examination distance Lid to a position closer than the examination distance Lid. Note that, in Example 2, the "Entire Configuration of Apparatus", "Configuration of Optical System", and "Configuration of Controller" have the same configurations as those in FIGS. 1, 2, and 3 of Example 1, and thus, illustration and description are omitted.

[Configuration and Flow of Actions of Process of Evaluating Adaptation Abilities to Stereoscopic Vision (FIG. 10)] The configuration and flow of actions of the process of evaluating the adaptation abilities to stereoscopic vision by the adaptation check portion 143 are described as follows with reference to the flowchart shown in FIG. 10. Note that steps S21, S22, S23, S24, S25, and S26 are the same processing steps corresponding to steps S1, S2, S3, S4, S5, and S6 in FIG. 4, respectively, and thus description thereof is omitted.

Step S27 is a parallax angle change processing step in which the subjective examination processing portion 144 performs a moving image visual target control of the main controller 141 that changes an initial parallax angle (0°) or a parallax angle of the previous time in a stepless manner with the examination distance (for example, 1 m) maintained. The visual target for the left eye 8L and the visual target for the right eye 8R of Example 2 are set as stereoscopic visual targets by the moving image on the display 41 (visual target presentation portion). Two asterisk marks 42L and 42R change the positions in a stepless manner from the position of the initial parallax angle (0°) to the position of the parallax angle (+5°). After that, the two asterisk marks 42L and 42R change the positions from the position of the parallax angle (+5°) to the position of the initial parallax angle (0°). Next, the two asterisk marks 42L and 42R change the positions in a stepless manner from the position of the initial parallax angle (0°) to the position of the final parallax angle (-5°).

Step S28 is a subjective response step in which the subjective examination processing portion 144 acquires a subjective response related to fusion of the left eye projection visual target and the right eye projection visual target from the subject, when the fusion is attempted with the left and right subject eyes EL and ER with respect to the stepless change in the convergence distance. The subjective examination processing portion 144, as the subjective response, for example, when the convergence distance changes in a stepless manner, acquires a response as to whether visual targets are seen as one visual target by fusion at predetermined time intervals is acquired. The change of the parallax angle may be temporarily stopped when the subjective response is prompted.

Step S29 is a final intersected parallax angle determination step in which the subjective examination processing portion 144 determines whether the parallax angle reaches to the final intersected parallax angle (±5°). While the subjective examination processing portion 144 determines NO in step S29, the process returns to step S27, and when the subjective examination processing portion 144 determines YES in step S29, the process proceeds to step S33. Note that, in step S29, time required for the parallax angle to reach the final intersected parallax angle (±5°) at the stepless change speed may be set to determine whether the set time elapses.

Steps S27 and S29 correspond to the convergence distance control step of prescribing a state in which the convergence distance from the positions of the left and right subject eyes EL and ER to the convergence position where the two lines of sight intersect each other by control that changes a parallax angle that is a difference in the positions of the two asterisk marks 82L and 82R reflected on the left and right subject eyes EL and ER. Steps S26 and S28 correspond to a subjective response acquisition step of acquiring a subjective response related to fusion from the subject when fusion is attempted by the left and right subject eyes EL and ER with respect to a change in the convergence distance.

Step S30 is a refractometry step in which the objective measurement portion 145 measures the eye characteristics of the left and right subject eyes EL and ER with respect to the change in the convergence distance by the objective measurement optical system and acquires the objective refraction value as the objective measurement information in time series. Step S31 is a refractometry value display step in which the objective measurement portion 145 displays objective refraction values (refractometry values) acquired in time series during the parallax angle changes in a stepless manner. Step S32 is a time-series refractometry value comparison step in which the objective measurement portion 145 compares refractometry values in time series acquired while the parallax angle changes in a stepless manner. These steps S30 to S32 correspond to the accommodation reaction amount measurement step of being executed in parallel with the control of the convergence distance, measuring the eye characteristics of the left and right subject eyes EL and ER by the objective measurement optical system when fusion is attempted by the left and right subject eyes EL and ER to the change of the convergence distance, and acquiring the objective refraction value as the objective measurement information.

Step S33 is a step in which the determination portion 146 determines adaptation abilities of the left and right subject eyes EL and ER to the stereoscopic vision based on the subjective response from the subjective examination processing portion 144 and the objective refraction value (time-series refractometry value) from the objective measurement portion 145. In step S33, the process proceeds to the end, when the determination portion 146 completes the determination of the adaptation abilities of the stereoscopic vision. This step S33 corresponds to a determination step of determining the adaptation abilities of the left and right subject eyes EL and ER to the stereoscopic vision based on the subjective response and the objective refraction value.

The flow of actions of each step is described as follows. In step S26, the process of FIG. 10 proceeds to step S27 to step S28 to step S29 after a subjective response related to fusion when the convergence distance and the examination distance are matched is acquired from the subject. In step S27, the subjective examination processing portion 144 controls the display speed of the moving image visual target of the main controller 141 to change consecutively the parallax angle from the initial parallax angle (0°) to the parallax angle in which the convergence distance becomes the closest (for example, +5°). In step S28, the subjective examination processing portion 144 acquires, from the subject, the subjective response related to the fusion when the parallax angle is switched to the parallax angle (for example, +0.5°). In step S29, the subjective examination processing portion 144 determines whether the parallax angle consecutively changes to the final parallax angle (for example, +5°) in the direction in which the convergence distance approaches, then changes to the initial parallax angle (for example, 0°) once, and then changes to the final parallax angle (for example, +5°) in the direction in which the convergence distance becomes the farthest.

In the process of FIG. 10, in step S29, while the subjective examination processing portion 144 determines that no parallax angle is changed to the final parallax angle (for example, ±5°), the flow from step S27 to step S28 to step S29 is repeated. In step S27, the subjective examination processing portion 144 performs the display speed control of the moving image visual target of the main controller 141 to change the parallax angle in a stepless manner. In step S28, while the parallax angle is changed in a stepless manner, the subjective examination processing portion 144 acquires a subjective response related to fusion at a predetermined time interval from the subject.

In the process of FIG. 10, in step S25, when the subjective examination processing portion 144 matches the convergence distance with the examination distance, the objective measurement portion 145 starts the objective measurement in parallel with the subjective examination process. In the objective measurement, during the subjective examination process, the flow proceeding to step S30 to step S31 to step S32 is repeated. If the subjective examination process ends (YES in step S29), the objective measurement ends in accordance with the end of the subjective examination process. In step S30, the objective measurement portion 145 acquires the objective refraction values (refractometry values) in time series as the objective measurement information by the refractometry. In step S31, the objective measurement portion 145 displays the acquired time-series refractometry value. In step S32, the objective measurement portion 145 compares the time-series refractometry value.

In the process of FIG. 10, in step S29, when the subjective examination processing portion 144 determines that the parallax angle reaches the final parallax angle (for example, - 5°), the process proceeds from step S29 to step S33. In step S33, the determination portion 146 determines adaptation abilities of the left and right subject eyes EL and ER to the stereoscopic vision based on the subjective response from the subjective examination processing portion 144 and the time-series objective refraction values from the objective measurement portion 145.

As described above, the flow of the processing action of evaluating the adaptation abilities to the stereoscopic vision is executed in parallel with the subjective examination process and the objective measurement and is a flow that merges with the objective measurement in step S33 when the subjective examination process ends. In step S33, for example, when a subjective response that the fusion by binocular vision is achieved is obtained irrelevant to the stepless change of the convergence distance and the time-series change of the objective refraction value is suppressed in a predetermined range, the determination portion 146 determines that the left and right subject eyes EL and ER have high adaptation abilities to stereoscopic vision. In step S33, for example, by changing the convergence distance in a stepless manner, when the subjective response that two visual targets are seen is obtained and no fusion by binocular vision is formed, or the time-series change of the objective refraction value exceeds the predetermined range, the determination portion 146 determines that the left and right subject eyes EL and ER have low adaptation abilities to stereoscopic vision. Furthermore, by obtaining time-series objective refraction values with respect to convergence stimulation, fluctuations in the objective refraction values can also be measured. For example, the determination portion 146 determines that the left and right subject eyes EL and ER have low adaptation abilities to stereoscopic vision when the frequency is analyzed, and the fluctuation range in a specific frequency band is large.

[Effect of Ophthalmic Examination Apparatus] (11) The subjective examination processing portion 144 prescribes a state in which the convergence distance L changes in a stepless manner in a distance range from a position farther than the examination distance Lid to a position closer than the examination distance Lid by changing the parallax (parallax angle) that is the difference in the positions of the two drawing patterns (asterisk marks 82L and 82R) reflected on the retinas of the left and right subject eyes EL and ER. The objective measurement portion 145 measures the eye characteristics of the left and right subject eyes EL and ER in a state where the convergence distance L changes in a stepless manner and acquires objective refraction values obtained in time series as objective measurement information. Therefore, the subjective examination processing portion 144 can give convergence stimulation that changes in a stepless manner to the left and right subject eyes EL and ER. In addition, the objective measurement portion 145 can measure fluctuations in the objective refraction values indicating fluctuations in the accommodation reaction amounts of the left and right subject eyes EL and ER with respect to convergence stimulation that changes in a stepless manner.

The ophthalmic examination apparatus 100 and the ophthalmic examination method (FIGS. 4 and 10) of Examples 1 and 2 are described above with reference to the drawings. However, the specific configurations of the ophthalmic examination apparatus and the ophthalmic examination method of the present disclosure are not limited to Examples 1 and 2, and modifications, additions, and the like of the design are allowed without departing from the gist of the invention according to each claim of the claims.

The ophthalmic examination apparatus 100 of Examples 1 and 2 is an example of an objective measurement machine with a subjective examination function by using an internal visual target integrally provided with a visual target presentation function, a phoropter function, and an Auto Ref/Keratometry function. However, the ophthalmic examination apparatus is not limited to the apparatus configuration examples of Examples 1 and 2. The ophthalmic examination apparatus may be an example that is configured with, for example, a combined apparatus of a visual target projection device that presents a real visual target and prescribes a state in which the convergence distance changes and a device having a phoropter function and an auto refractometry function.

An example in which the ophthalmic examination apparatus 100 in Examples 1 and 2 changes the parallax angle (parallax distance) for determining the left and right direction position of one asterisk marks 82L and 82R as the drawing pattern on each of the visual target frames 81L and 81R is provided. The control that is executed by the ophthalmic examination apparatus is not limited to the control examples of the parallax angle (parallax distance) by using the visual target for the left eye and the visual target for the right eye by one drawing pattern of Examples 1 and 2. For example, the ophthalmic examination apparatus may be an example of changing the parallax angle (parallax distance) by using a precise stereoscopic visual target in which a plurality of drawing patterns are arranged in the visual target frame.

An example in which the determination portion 146 in Examples 1 and 2 determines the adaptation abilities of the left and right subject eyes EL and ER to stereoscopic vision based on the subjective response acquired from the subject by the subjective examination processing portion 144 and the objective refraction value acquired by the objective measurement portion 145 is provided. However, the determination portion is not limited to the examples of the determination portion in Examples 1 and 2. For example, the determination portion may be an example of determining the adaptation abilities of the left and right subject eyes to stereoscopic vision based on only the objective refraction value.

Example 1 provides an example in which the subjective examination processing portion 144 performs the control that changes the parallax angle in a stepwise manner and acquires the average objective refraction value obtained by averaging the objective refraction values obtained a predetermined number of times in each step. Example 2 provides an example in which the subjective examination processing portion 144 performs the control that changes the parallax angle in a stepless manner and acquires the objective refraction value obtained in time series. However, the subjective examination processing portion is not limited to Examples 1 and 2. The subjective examination processing portion may be an example of performing the control that changes the parallax angle in a stepwise manner and acquires the objective refraction value obtained in time series regardless of the step control of the parallax angle.

The ophthalmic examination apparatus and the ophthalmic examination method of the present disclosure are also acceptable for the following points. (a) The range of the parallax angle may be determined on the assumption of, for example, a 3D video to be viewed or an actual value on a head mounted display. Accordingly, the adaptation abilities to a specific stereoscopic image can be determined. (b) The anterior segment image may be consecutively acquired during the measurement of the objective refraction value, so that alignment may be automatically performed when the alignment is deviated. (c) In the determination of the adaptation abilities of the left and right subject eyes EL and ER to the stereoscopic vision, low adaptation abilities to the stereoscopic vision may be determined when the anterior segment images may be acquired in time series or at the timing when the parallax angle changes, and a near vision reaction in which the pupil contracts, for example, when the convergence distance becomes near occurs by a predetermined amount or more. (d) In the determination of the adaptation abilities of the left and right subject eyes EL and ER to the stereoscopic vision, an adaptation coefficient to the stereoscopic vision may be calculated by combining a plurality of determination criteria.

## Claims

1. An ophthalmic examination apparatus (100) comprising:
a visual target projection system (4) including a visual target presentation portion (41) that presents a fixed visual target to left and right subject eyes (EL, ER);
an objective measurement optical system (6, 7) that objectively measures eye characteristics of the left and right subject eyes (EL, ER); and
a controller (140) that controls each portion of the apparatus (100), wherein
the fixed visual target is a visual target for the left eye (8L) and a visual target for the right eye (8R) that are to be respectively projected to the left and right subject eyes (EL, ER) and have a same drawing pattern in positions where up and down direction positions to visual target flames (81L, 81R) are the same,
the visual target presentation portion (41) presents the visual target for the left eye (8L) and the visual target for the right eye (8R) to visual target positions that are separated from the left and right subject eyes (EL, ER) by a certain examination distance,
the visual target projection system (4) prescribes a state in which a convergence distance from positions of the left and right subject eyes (EL, ER) to a convergence position where two lines of sight intersect each other is changed by changing parallax that is difference in positions of the two drawing patterns reflected on retinas of the left and right subject eyes (EL, ER), and
the controller (140) measures the eye characteristics of the left and right subject eyes (EL, ER) by the objective measurement optical system (6, 7) and acquires an objective refraction value as objective measurement information when fusion is attempted with the left and right subject eyes (EL, ER) with respect to a change in the convergence distance.

2. The ophthalmic examination apparatus (100) according to claim 1, wherein
the ophthalmic examination apparatus (100) is an objective measurement machine with a subjective examination function,
the controller (140) includes an adaptation check portion (143) that evaluates adaptation abilities of the left and right subject eyes (EL, ER), and the adaptation check portion (143) includes a subjective examination processing portion (144) and an objective measurement portion (145),
the subjective examination processing portion (144) presents the visual target for the left eye (8L) and the visual target for the right eye (8R) to the visual target positions that are separated from the left and right subject eyes (EL, ER) by the certain examination distance and prescribes a state in which the convergence distance from the positions of the left and right subject eyes (EL, ER) to the convergence position where the two lines of sight intersect each other is changed by control that changes the parallax that is the difference in the positions of the two drawing patterns reflected on the retinas of the left and right subject eyes (EL, ER), and
the objective measurement portion (145) execute a process in parallel with a process in the subjective examination processing portion (144), measures the eye characteristics of the left and right subject eyes (EL, ER), when fusion is attempted with the left and right subject eyes (EL, ER) to the change of the convergence distance with the objective measurement optical system (6, 7), and acquires the objective refraction value as objective measurement information.

3. The ophthalmic examination apparatus (100) according to claim 2, wherein
the visual target for the left eye (8L) and the visual target for the right eye (8R) are set to stereoscopic visual targets provided with the parallax by differentiating the positions of the displayed drawing patterns in the left and right direction to differentiate the left and right direction positions of the two drawing patterns to the visual target flames (81L, 81R), and
the subjective examination processing portion (144) sets the control that changes the parallax to control that changes a parallax angle or a parallax distance when the fusion of the two drawing patterns is attempted in binocular vison.

4. The ophthalmic examination apparatus (100) according to claim 2, wherein
the subjective examination processing portion (144) acquires a subjective response from a subject when the fusion is attempted by the left and right subject eyes (EL, ER) with respect to the change in the convergence distance, and
the adaptation check portion (143) includes, in addition to the subjective examination processing portion (144) and the objective measurement portion (145), a determination portion (146) that determines adaptation abilities of the left and right subject eyes (EL, ER) to a stereoscopic vision based on the subjective response from the subject acquired with the subjective examination processing portion (144) and the objective refraction value acquired with the objective measurement portion (145).

5. The ophthalmic examination apparatus according to claim 4, wherein
the determination portion (146) determines that the left and right subject eyes (EL, ER) have high adaptation abilities to stereoscopic vision when a positive subjective response to the fusion in binocular vision is obtained with respect to the change in the convergence distance, and a change width of the objective refraction value is suppressed to a predetermined range.

6. The ophthalmic examination apparatus (100) according to claim 4, wherein
the determination portion (146) determines that the left and right subject eyes (EL, ER) have low adaptation abilities to stereoscopic vision when a negative subjective response to the fusion in binocular vision is obtained with respect to the change in the convergence distance, and a change width of the objective refraction value exceeds a predetermined range.

7. The ophthalmic examination apparatus (100) according to claim 2, wherein
the adaptation check portion (143) includes, in addition to the subjective examination processing portion (144) and the objective measurement portion (145), a determination portion (146) that determines the adaptation abilities of the left and right subject eyes (EL, ER) to stereoscopic vision based on the objective refraction value acquired with the objective measurement portion (145), and
the determination portion (146) measures an amplitude that is a fluctuation range of a refractive value characteristic of the objective refraction value acquired with the objective measurement portion (145) with respect to a time axis and determines that the left and right subject eyes (EL, ER) belong to a fatigue group, when a fluctuation frequency of the refractive value characteristic is a low frequency band, and the amplitude is a determination threshold or more.

8. The ophthalmic examination apparatus (100) according to any one of claims 2-7, wherein
the subjective examination processing portion (144) prescribes a state in which the convergence distance gradually changes in a distance range from a position farther than the examination distance to a position closer than the examination distance by control that gradually changes the parallax that is the difference in the positions of the two drawing patterns reflected on the retinas of the right and left subject eyes (EL, ER), and
the objective measurement portion (145) measures the eye characteristics of the left and right subject eyes (EL, ER) a predetermined number of times in each step of the convergence distance and acquires an average objective refraction value obtained by averaging objective refraction values obtained a predetermined number of times in each step as objective measurement information.

9. The ophthalmic examination apparatus (100) according to any one of claims 2-7, wherein
the subjective examination processing portion (144) prescribes a state in which the convergence distance gradually changes in a distance range from a position farther than the examination distance to a position closer than the examination distance by control that changes in a stepless manner the parallax that is the difference in the positions of the two drawing patterns reflected on the retinas of the right and left subject eyes (EL, ER), and
the objective measurement portion (145) measures the eye characteristics of the left and right subject eyes (EL, ER) in a state in which the convergence distance changes in a stepless manner and acquires an objective refraction value obtained in time series as objective measurement information.

10. An ophthalmic examination method in which
a visual target projection system (4) that includes a visual target presentation portion that presents a fixed visual target to left and right subject eyes (EL, ER),
an objective measurement optical system (6, 7) that objectively measures eye characteristics of the left and right subject eyes (EL, ER), and
a controller (140) that controls each portion of apparatus are included, the method causes the controller (140) to perform:
a complete correction prescription step of assuming the left and right subject eyes (EL, ER) as a state in which a complete correction value in subjective examination is prescribed;
a visual target presentation step of presenting a visual target for the left eye (8L) and a visual target for the right eye (8R) having a same pattern in the visual target presentation portion at visual target positions separated from the left and right subject eyes (EL, ER) by a certain examination distance following the prescription of the complete correction value;
a convergence distance control step of prescribing a state in which a convergence distance from positions of the left and right subject eyes (EL, ER) to a convergence position where the two lines of sight intersect each other is changed by control that changes parallax that is difference in positions of the two drawing patterns reflected on retinas of the left and right subject eyes (EL, ER);
an accommodation reaction amount measurement step of being executed in parallel with the control of the convergence distance and measuring the eye characteristics of the left and right subject eyes (EL, ER) with the objective measurement optical system (6, 7) when fusion is attempted with the left and right subject eyes (EL, ER) to the change of the convergence distance to acquire an objective refraction value as objective measurement information; and
a determination step of determining adaptation abilities of the left and right subject eyes (EL, ER) to stereoscopic vision based on the objective refraction value.

11. The ophthalmic examination method according to claim 10, further comprising
a subjective response acquisition step of acquiring a subjective response related to the fusion from a subject when the fusion is attempted to a change in the convergence distance with the left and right subject eyes (EL, ER), wherein
the determination step determines adaptation abilities of the left and right subject eyes (EL, ER) to stereoscopic vision based on the subjective response and the objective refraction value.
